(19) **Europäisches Patentamt / European Patent Office / Office européen des brevets**

(11) **EP 1 984 696 B1**

(12) **FASCICULE DE BREVET EUROPEEN**

(45) Date de publication et mention
de la délivrance du brevet:
**08.07.2009 Bulletin 2009/28**

(21) Numéro de dépôt: **07726410.9**

(22) Date de dépôt: **16.02.2007**

(51) Int Cl.:
*G01C 21/16* (2006.01)     *G06F 3/00* (2006.01)
*A61B 5/103* (2006.01)

(86) Numéro de dépôt international:
**PCT/EP2007/051522**

(87) Numéro de publication internationale:
**WO 2007/093641 (23.08.2007 Gazette 2007/34)**

(54) **DISPOSITIF DE CAPTURE DE MOUVEMENT ET PROCEDE ASSOCIE**

BEWEGUNGSERFASSUNGSVORRICHTUNG UND ENTSPRECHENDES VERFAHREN

MOTION CAPTURE DEVICE AND ASSOCIATED METHOD

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **17.02.2006 FR 0601410**

(43) Date de publication de la demande:
**29.10.2008 Bulletin 2008/44**

(73) Titulaire: **COMMISSARIAT A L'ENERGIE ATOMIQUE**
**75015 Paris (FR)**

(72) Inventeurs:
• **CARITU, Yanis**
**F-38134 Saint Joseph de Riviere (FR)**

• **DAVID, Dominique**
**F-38640 Claix (FR)**
• **GODIN, Christelle**
**F-38190 Brignoud (FR)**

(74) Mandataire: **Ilgart, Jean-Christophe**
**BREVALEX**
**3, rue du Docteur Lancereaux**
**75008 Paris (FR)**

(56) Documents cités:
**EP-A- 1 593 931        WO-A-20/05016143**
**FR-A1- 2 838 185        FR-A1- 2 860 700**
**US-B2- 6 820 025**

EP 1 984 696 B1

**Description**

Domaine technique et art antérieur

L'invention concerne un dispositif de capture de mouvement et le procédé de capture de mouvement associé. L'invention concerne également un dispositif de reproduction de mouvement et le procédé de reproduction de mouvement associé.

Un dispositif de capture de mouvement d'une structure est un dispositif qui mesure des grandeurs aptes à décrire, par traitement, le mouvement de la structure. La structure peut être, par exemple, une personne ou un robot en déplacement ou non.

La capture du mouvement humain est une technique très utilisée dans de nombreuses applications: analyse biomécanique, télémanipulation, animation de personnage, ergonomie, etc.

Une première catégorie de dispositifs de capture de mouvement est constituée de dispositifs qui comprennent deux parties distinctes : une première partie est placée sur l'objet en mouvement et une deuxième partie est fixe par rapport au mouvement de l'objet. Dans cette première catégorie, on distingue principalement les systèmes optiques, les systèmes électromagnétiques et les systèmes à ultrasons. Ces dispositifs sont performants en termes de précision. Ils présentent toutefois un certain nombre d'inconvénients. Il est ainsi nécessaire d'installer du matériel à la fois sur l'objet et dans l'environnement de l'objet. Dans tous les cas, ces systèmes ont une portée réduite (en rapport à la portée de la source physique) et une phase d'installation et de calibration assez longue. Leur coût est également très élevé.

La technologie probablement la plus utilisée à l'heure actuelle est basée sur l'optique, comme cela est décrit, par exemple, dans les demandes de brevet US 2003/0215130 A1 et US 2005/00883333 A1. Ces systèmes permettent de reconstruire les mouvements du corps à partir d'images vues par des caméras placées tout autour de la scène où se déroule l'action. Sur l'objet en mouvement sont disposées des marqueurs très visibles pour les caméras. Un traitement permet de fournir la position 3D (3D pour « à trois dimensions ») de chaque marqueur par le principe de stéréoscopie. Malgré cela, les problèmes d'occlusion optique sont nombreux, ce qui rend important le nombre minimal de caméras utilisé. Certains auteurs proposent de réduire ce type de désavantages, comme cela apparaît, par exemple dans le document intitulé « Skeleton-Based Motion Capture for Robust Reconstruction of Human Motion » (L.Herda ; P.Fua ; R.Pl ankers ; R.Boulic ; D.Thalmann, Computer Graph Lab (LIG), EPFL - web 01/2000). D'autres auteurs proposent des méthodes de traitement basées sur la silhouette extraite à partir d'une seule caméra en lui associant le modèle de l'objet en mouvement (cf. « Marker-free Kinematic Skeleton Estimation from Sequences of Volume Data » C.Theobalt ; E.Aguiar ; M.Magnor ; H.Theisel ; H-P.Seidel ; MPI Informatik).

Les systèmes basés sur l'électromagnétisme reconstruisent les angles et les positions des capteurs disposés sur l'objet.

Les systèmes à ultrasons, de même que les systèmes optiques, retrouvent les positions des émetteurs. Ces deux technologies souffrent de la même limitation dans l'espace que la technologie à base de caméra.

Une seconde catégorie de dispositifs concerne des dispositifs en un seul bloc disposé sur le mobile. C'est le cas des dispositifs exosquelette. Ces dispositifs permettent de s'affranchir de la limitation du volume de capture mais sont contraignants puisqu'ils sont constitués de bras articulés mécaniques disposés sur la structure ou la personne. La reconstruction du mouvement utilise des mesures d'angle et non de position entre les segments des membres articulés.

Plus récemment, des systèmes basés sur un principe assez ancien (le principe des centrales inertielles) ont vu le jour à des échelles plus petites que les échelles traditionnelles, typiquement de quelques centimètres de côté (cf. le brevet US 6 162 191). Ces dispositifs constitués de capteurs de vitesse angulaire (gyromètres) sont placés sur le mobile ou la personne en mouvement. Les capteurs de vitesse angulaire fournissent les angles des segments en rotation à condition d'intégrer une fois la mesure, ce qui occasionne une dérive. Aux gyromètres sont parfois associés des accéléromètres, voire des magnétomètres, de sorte que, dès que le mouvement est plus lent, la mesure de ces derniers, s'appuyant sur les champs magnétiques et gravitationnels terrestres recalent l'estimation de l'orientation, annulant ainsi la dérive. La capture de mouvements rapides reste néanmoins un problème si les accélérations demeurent, car le recalage n'intervient plus. En outre, les gyromètres sont des capteurs encore difficiles à mettre en oeuvre, assez chers et présentant également une certaine sensibilité aux accélérations.

Une autre approche (cf. le brevet US 6 820 025) consiste à juxtaposer aux segments articulés des capteurs d'angle comportant des gyromètres pour reconstruire le mouvement.

La demande de brevet français FR 2 838 185 décrit un dispositif de capture de l'orientation d'un solide qui se meut dans un repère de référence. Le dispositif de capture de mouvement fournit, à partir de mesures issues de capteurs axiaux ou vectoriels placés sur le solide, au moins un angle d'orientation $\theta$ que fait le repère mobile du solide dans le repère de référence. Les capteurs utilisés sont préférentiellement un magnétomètre et un accéléromètre. Il existe alors une équation (1) entre les mesures M, le champ de gravitation G exprimé dans le repère de référence, le champ magnétique H exprimé dans le repère de référence et l'angle d'orientation $\theta$ :

$$M = F(\theta, G, H) \qquad (1)$$

Les mesures M des grandeurs physiques qui sont effectuées respectivement par l'accéléromètre et par le magnétomètre sont ainsi modélisées comme une fonction F qui traduit la rotation $\theta$ du repère attaché au solide par rapport au repère fixe dans lequel évolue le solide.

L'angle d'orientation $\theta$ est déduit de l'équation (1) par l'équation (2) suivante :

$$\theta = F^{-1}(M, G, H) \qquad (2).$$

Si le mouvement est accéléré, une nouvelle équation (3) décrit le système, à savoir :

$$M = F(\theta, a, G, H) \qquad (3).$$

Les inconnues $\theta$ et a forment alors un espace de dimension élevée qui interdit, de façon pratique, une inversion de la fonction F. Il n'est alors pas possible d'extraire les inconnues $\theta$ et a de l'équation (3). Sans information supplémentaire, le dispositif ne permet donc pas la mesure des angles d'orientation dès lors que le mobile est accéléré ou, du moins, dès lors que l'accélération du mobile ne peut pas être négligée. Ceci représente un inconvénient.

L'invention ne présente pas les inconvénients des dispositifs mentionnés ci-dessus.

Exposé de l'invention

En effet, l'invention concerne un dispositif de capture de mouvement d'une structure constituée de N segments solides successifs articulés les uns par rapport aux autres à partir d'un segment de rang 1 jusqu'à un segment de rang N, N étant un nombre entier supérieur ou égal à 2, le segment de rang n (n = 2, ..., N) étant articulé avec le segment de rang n-1 au niveau d'un point d'articulation $p_n$, **caractérisé en ce qu'**il comprend :

- des premiers moyens qui délivrent une information apte à restituer un vecteur d'accélération absolue $\vec{a}_1$ d'un point du segment de rang 1 dans un repère de référence, à des instants successifs $t_k$, k étant un nombre entier supérieur ou égal à 1,
- des seconds moyens de mesure fixés sur le segment de rang 1 et qui délivrent, à chaque instant $t_k$, une mesure représentative d'un vecteur orientation $\vec{\Theta}_1$ du segment de rang 1 dans le repère de référence, et
- des moyens de mesure supplémentaires fixés sur chaque segment de rang n (n = 2, ..., N) et qui délivrent, à chaque instant $t_k$, une mesure représentative d'un vecteur orientation $\vec{\Theta}_n$ du segment de rang n.

Selon une caractéristique supplémentaire de l'invention, les seconds moyens de mesure et les moyens de mesure supplémentaires sont constitués d'un accéléromètre et d'un capteur qui délivre une mesure d'un champ physique uniforme présent dans l'espace où se meut la structure et de direction connue dans le repère de référence.

Selon une autre caractéristique supplémentaire de l'invention, les seconds moyens de mesure et les moyens de mesure supplémentaires comprennent, en outre, au moins un axe gyrométrique.

Selon encore une autre caractéristique supplémentaire de l'invention, le capteur qui délivre une mesure d'un champ physique uniforme de direction connue dans le repère de référence est un magnétomètre.

Selon encore une autre caractéristique supplémentaire de l'invention, le capteur qui délivre une mesure d'un champ physique uniforme de direction connue dans le repère de référence est une cellule photoélectrique.

Selon encore une autre caractéristique supplémentaire de l'invention, les premiers moyens sont constitués d'un mesureur de vitesse de sorte que l'information apte à restituer un vecteur d'accélération absolue d'un point du segment de rang 1 est la vitesse du point.

Selon encore une autre caractéristique supplémentaire de l'invention, les premiers moyens sont constitués d'un mesureur de position de sorte que l'information apte à restituer un vecteur d'accélération absolue d'un point du segment de rang 1 est la position du point.

L'invention concerne également un dispositif de reproduction de mouvement d'une structure constituée de N segments solides successifs articulés les uns par rapport aux autres à partir d'un segment de rang 1 jusqu'à un segment de rang N, N étant un nombre entier supérieur ou égal à 2, le segment de rang n (n = 2, ..., N) étant articulé avec le segment de rang n-1 au niveau d'un point d'articulation $p_n$, **caractérisé en ce qu'**il comprend :

- un dispositif de capture de mouvement selon l'invention dans lequel les moyens de mesure supplémentaires d'un segment de rang n sont positionnés à proximité du point d'articulation $p_n$ de telle sorte que la distance qui sépare les moyens de mesure supplémentaire d'un segment de rang n du point d'articulation $p_n$ est considérée comme nulle, et
- des moyens de calcul qui calculent, à chaque instant $t_k$ :

a) le vecteur d'accélération absolue $\vec{a}_1$ dans le repère de référence, à partir de l'information délivrée par les premiers moyens,
b) le vecteur d'orientation $\vec{\Theta}_1$ du segment de rang 1 dans le repère de référence, à partir du vecteur d'accélération absolue $\vec{a}_1$ et de la mesure représentative du vecteur orientation $\vec{\Theta}_1$ du segment de rang 1 ;
c) un vecteur d'accélération $\vec{a}_n$ $(n \geq 2)$ du point d'articulation $p_n$ dans le repère de référence, à partir de l'équation :

$$\vec{a}_n = \vec{a}_{n-1} + \left(\frac{d\vec{\omega}_{n-1}}{dt}\right)\Lambda \overrightarrow{L_{n-1}} + \vec{\omega}_{n-1}\Lambda\left(\vec{\omega}_{n-1}\Lambda\overrightarrow{L_{n-1}}\right)$$

où $\overrightarrow{\omega_n}$ = $d(\vec{\Theta}_n)/dt$ et $\overrightarrow{L_n}$ étant un vecteur orienté du point d'articulation $p_{n-1}$ vers le point d'articulation $p_n$ et dont le module a pour valeur la distance qui sépare le point d'articulation $p_n$ du point d'articulation $p_{n-1}$; et
d) le vecteur d'orientation $\vec{\Theta}_n$ $(n \geq 2)$ du segment de rang n à partir du vecteur d'accélération $\vec{a}_n$ et de la mesure représentative de l'orientation du segment de rang n.

L'invention concerne également un dispositif de reproduction de mouvement d'une structure constituée de N segments solides successifs articulés les uns par rapport aux autres à partir d'un segment de rang 1 jusqu'à un segment de rang N, N étant un nombre entier supérieur ou égal à 2, le segment de rang n (n = 2, ..., N) étant articulé avec le segment de rang n-1 au niveau d'un point d'articulation $p_n$, **caractérisé en ce qu'**il comprend :

- un dispositif de capture de mouvement selon l'invention dans lequel les moyens de mesure supplémentaires d'un segment de rang n sont distants du point d'articulation $p_n$, et
- des moyens de calcul qui calculent, à chaque instant $t_k$ :

a) le vecteur d'accélération absolue $\vec{a}_1$ dans le repère de référence, à partir de l'information délivrée par les premiers moyens,
b) le vecteur d'orientation $\vec{\Theta}_1$ du segment de rang 1 dans le repère de référence, à partir du vecteur d'accélération absolue $\vec{a}_1$ et de la mesure représentative du vecteur orientation $\vec{\Theta}_1$ du segment de rang 1 ;
c) un vecteur d'accélération $\vec{a}_n$ $(n \geq 2)$ du point d'articulation $p_n$ dans le repère de référence, à partir de l'équation :

$$\vec{a}_n = \vec{a}_{n-1} + \left(\frac{d\vec{\omega}_{n-1}}{dt}\right)\Lambda \overrightarrow{L_{n-1}} + \vec{\omega}_{n-1}\Lambda\left(\vec{\omega}_{n-1}\Lambda\overrightarrow{L_{n-1}}\right)$$

où $\overrightarrow{\omega_n}$ = $d(\vec{\Theta}_n)/dt$, $\overrightarrow{L_n}$ étant un vecteur orienté du point d'articulation $p_{n-1}$ vers le point d'articulation $p_n$ et dont le module a pour valeur la distance qui sépare le point d'articulation $p_n$ du point d'articulation $p_{n-1}$; et
d) le vecteur d'orientation $\vec{\Theta}_n$ $(n \geq 2)$ et un vecteur d'accélération $\vec{b}_n$ du point de mesure des moyens de mesure supplémentaire fixés sur le segment de rang n à partir du vecteur d'accélération $\vec{a}_n$, de la mesure représentative $(M_n)$ de l'orientation du segment de rang n, et des vecteurs d'orientation du segment de rang n à au moins deux instants qui précèdent l'instant $t_k$, avec $\vec{b}_n$ tel que :

$$\vec{b}_n = \vec{a}_n + \left(\frac{d\vec{\omega}_n}{dt}\right)\wedge \vec{D}_n + \vec{\omega}_n \wedge\left(\vec{\omega}_n \wedge \vec{D}_n\right)$$

où $\vec{D}_n$ est un vecteur orienté du point d'articulation $p_n$ vers les moyens de mesure supplémentaires du segment de rang n et dont le module est sensiblement égal à la distance qui sépare le point d'articulation $p_n$ des moyens de mesure supplémentaires du segment de rang n.
Selon une caractéristique supplémentaire de l'invention, des moyens de transmission radioélectrique transmettent des signaux électriques élémentaires représentatifs des mesures délivrées par les premiers moyens de mesure et les seconds moyens de mesure vers les moyens de calcul.

Selon encore une caractéristique supplémentaire de l'invention, les moyens de transmission radioélectrique comprennent une unité intermédiaire qui reçoit les signaux électriques élémentaires et qui réémet un signal électrique représentatif des signaux électriques élémentaires vers les moyens de calcul.

Selon encore une caractéristique supplémentaire de l'invention, des moyens de mémorisation mémorisent les mesures délivrées par les premiers moyens de mesure et les seconds moyens de mesure.

Selon encore une caractéristique supplémentaire de l'invention, les moyens de mémorisation sont placés sur la structure.

L'invention concerne également :

- un procédé de capture de mouvement conforme à la revendication indépendante 14,
- un procédé de reproduction de mouvement conforme à la revendication indépendante 18, et
- un procédé de reproduction de mouvement conforme à la revendication indépendante 19.

Un dispositif élémentaire de mesures selon l'invention est constitué de deux types de capteurs dont au moins un est un accéléromètre.

Préférentiellement, un dispositif élémentaire de mesures est réalisé à l'aide d'un dispositif de capture de mouvement de rotation de solide tel que celui décrit dans la demande de brevet français publiée sous la référence FR 2 838 185 et déposée, au nom de la Demanderesse, en date du 5 avril 2002. Un dispositif élémentaire de mesures est ainsi constitué d'un couple (accéléromètre, capteur X).

Par capteur X, il faut entendre n'importe quel capteur qui fournit une mesure d'un champ physique uniforme présent dans l'espace où évolue le mobile, champ physique dont la direction est connue dans le repère de référence ou qui est mesuré dans une position de référence. Les seules contraintes concernant le capteur X sont, d'une part, que le capteur ne doit pas être sensible aux accélérations et, d'autre part, que la direction du champ physique mesuré soit différente de la verticale. Le capteur X peut ainsi être un magnétomètre qui mesure la direction du champ magnétique terrestre. Le capteur X peut également être une cellule photoélectrique dont la mesure est celle de l'intensité lumineuse qui arrive sur la cellule. Si, par exemple, la source d'éclairement est le soleil et que l'on connaît la date, l'heure, la longitude et la latitude lors de la mesure de l'intensité lumineuse, on sait prédire l'angle d'incidence du rayon solaire dans un repère absolu et, en conséquence, la mesure est modulée en fonction de l'angle que fait le dispositif par rapport à la direction du rayon solaire. C'est donc également une autre façon de mesurer un angle. Le capteur X peut encore être constitué de un ou de plusieurs axes gyrométriques qui viennent compléter la mesure de l'accéléromètre.

Les premiers moyens qui délivrent une information apte à restituer un vecteur d'accélération absolue $\vec{a}_1$ d'un point du segment de rang 1 peuvent être réalisés par un système de mesures locales. Un simple accéléromètre ne convient pas si on ne dispose pas de moyens pour compenser l'accélération de la pesanteur. Dans le cas concret de la mesure du mouvement d'une personne, le système de mesure locale peut être avantageusement placé au centre de masse ou à proximité du centre de masse du corps de la personne (à la ceinture, par exemple).

Le système de mesures locales peut être, par exemple, un dispositif de type GPS (GPS pour « Global Positioning System ») associé à un dérivateur. Le dispositif GPS permet de connaître à tout instant la position de l'élément qui le porte et un dérivateur, en dérivant deux fois la donnée de position, détermine l'accélération absolue dans le repère géographique.

Le système de mesures locales peut également être réalisé à l'aide d'un dispositif de radio localisation associé à un dérivateur. Les dispositifs de radio localisation nécessitent l'utilisation de balises (radar ULB (ULB pour « Ultra Large Bande », balise optique, etc.). Les dispositifs de radio localisation font donc perdre le caractère autonome du système de mesures locales. Ils se révèlent toutefois d'utilisation très avantageuse lors de suivis de mouvements dans une enceinte où des balises sont préalablement positionnées. L'utilisation de systèmes radio présente également le double avantage de la transmission des données et de la mesure de position (c'est particulièrement le cas des dispositifs ULB). De même que dans le cas du dispositif GPS, la mesure de position délivrée par le dispositif de radio localisation est dérivée deux fois pour obtenir la mesure d'accélération.

Une mesure de pression orientée (tube) est directement corrélée à la vitesse d'un corps dans l'air. Il est ainsi possible de déterminer, selon trois axes, le vecteur vitesse d'un segment sur lequel est fixé un mesureur de pression. En dérivant une fois la mesure de vitesse, on obtient l'accélération.

Le dispositif de capture de mouvement peut avantageusement être « dynamique » au niveau de la structure hiérarchique de la structure en mouvement. Dans le cas, par exemple, d'une structure humanoïde (personne ou robot), cela signifie que le (les) système(s) de mesure locale ML peut (peuvent) être placé(s) indifféremment au niveau du pied, de la main, de la taille, etc., ou de tout autre partie du corps assimilable à un élément rigide.

Dans d'autres modes de réalisation de l'invention, les premiers moyens qui délivrent une information apte à restituer un vecteur d'accélération absolue $\vec{a}_1$ d'un point du segment de rang 1 ne sont pas des moyens de mesures.

Dans le cas où il est connu qu'un point d'un segment est fixe dans le repère de référence, il est en effet inutile d'effectuer une mesure d'accélération sur ce segment. Ce segment peut alors avantageusement être choisi comme étant le segment de rang 1. Les premiers moyens qui délivrent l'information apte à restituer un vecteur d'accélération absolue $\vec{a_1}$ d'un point du segment de rang 1 peuvent alors être, par exemple, des moyens de stockage qui ont la connaissance de la position fixe occupée par un point du segment de rang 1 dans le repère de référence.

A titre d'exemple non limitatif, dans la suite de la description, les premiers moyens qui délivrent une information apte à restituer un vecteur d'accélération absolue $\vec{a_1}$ sont des moyens de mesure ML fixés sur le segment de rang 1. Les moyens de mesure ML seront considérés comme superposés aux seconds moyens de mesure $MD_1$ qui sont également fixés sur le segment de rang 1. Dans un cas plus général, les moyens de mesure ML et $MD_1$ sont distants les uns des autres, la position des moyens de mesure ML étant alors assimilable à un point d'articulation virtuel entre lé segment de rang 1 et un segment virtuel de rang zéro.

Un dispositif de mesure MD peut caractériser un état de repos. La variance des signaux délivrés par le dispositif MD est alors inférieure à un seuil. Dès lors qu'un état de repos est détecté en un point, il existe une très forte probabilité pour que ce point soit au repos dans le repère fixe de référence (en effet, bien qu'un mouvement rectiligne uniforme donne le même résultat qu'un état de repos, un tel mouvement est peu probable et difficile à maintenir). Dans le cas d'un repos détecté, l'accélération de la structure est nulle et l'état de repos peut être détecté.

Toutefois, il y a des cas où une articulation est au repos dans un mouvement particulier. C'est le cas, par exemple, de la marche où chaque pied se trouve être momentanément au repos de façon alternée. Dans ce cas, le procédé de l'invention s'applique de façon que le segment de rang 1 soit, alternativement, le pied droit ou le pied gauche.

Dans la suite de la description, l'invention sera décrite pour la capture et la reproduction du mouvement d'une structure articulée constituée d'une succession de segments. Cependant, il est clair que l'invention s'applique également à tout corps solide non articulé et de forme quelconque (celui-ci peut alors être identifié au segment de rang 1 de la structure articulée décrite) ou encore à une structure articulée complexe constituée de plusieurs ensembles de segments articulés.

Brève description des dessins

D'autres caractéristiques et avantages de l'invention apparaîtront à la lecture d'un mode de réalisation préférentiel fait en référence aux figures jointes parmi lesquelles :

- la figure 1 représente, de manière symbolique, un exemple de structure articulée concernée par le dispositif de capture de mouvement de l'invention ;
- la figure 2 représente un exemple de dispositif de capture de mouvement selon l'invention dans le cas d'une structure à quatre segments articulés ;
- la figure 3 représente deux segments articulés successifs munis d'un dispositif de capture de mouvement selon l'invention ;
- la figure 4A représente des étapes essentielles d'un cas particulier du procédé de traitement de mesures mis en oeuvre dans le cadre de l'invention ;
- la figure 4B représente, dans le cas général, des étapes essentielles du procédé de traitement de mesures mis en oeuvre dans le cadre de l'invention ;
- la figure 5A représente un organigramme détaillé d'une étape essentielle du procédé de traitement de mesures représenté en figure 4A ;
- la figure 5B représente un organigramme détaillé d'une étape essentielle du procédé de traitement de mesures représenté en figure 4B ;
- la figure 6A illustre, de façon symbolique, dans le cas particulier mentionné ci-dessus, l'évolution au cours du temps des données d'accélération et d'orientation obtenues pour les différents segments d'une structure à cinq segments articulés ;
- la figure 6B illustre, dans le cas général, les résultats de calcul, de proche en proche, des données d'accélération et d'orientation obtenues pour les différents segments d'une structure à segments articulés ;
- les figures 7A et 7B représentent deux modes de réalisation d'un dispositif de reproduction de mouvement selon l'invention.

Sur toutes les figures, les mêmes références représentent les mêmes éléments.

Description détaillée de modes de mise en oeuvre de l'invention

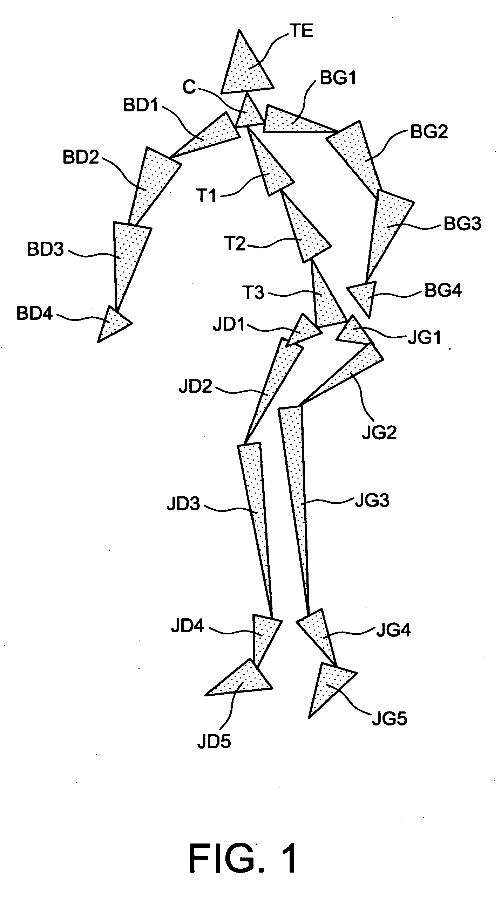

La figure 1 représente un exemple de structure articulée concernée par le dispositif de capture de mouvement de l'invention.

EP 1 984 696 B1

La structure, par exemple un corps humain ou un robot humanoïde, est décomposée en un ensemble de segments qui sont autant d'éléments solides articulés les uns par rapport aux autres. L'ensemble des segments se décompose ainsi en un segment de tête TE, un segment de cou C, un ensemble de segments de tronc T1, T2, T3, un ensemble de segments de bras gauche BG1, BG2, BG3, BG4, un ensemble de segments de bras droit BD1, BD2, BD3, BD4, un ensemble de segments de jambe gauche JG1, JG2, JG3, JG4, JG5 et un ensemble de segments de jambe droite JD1, JD2, JD3, JD4, JD5.

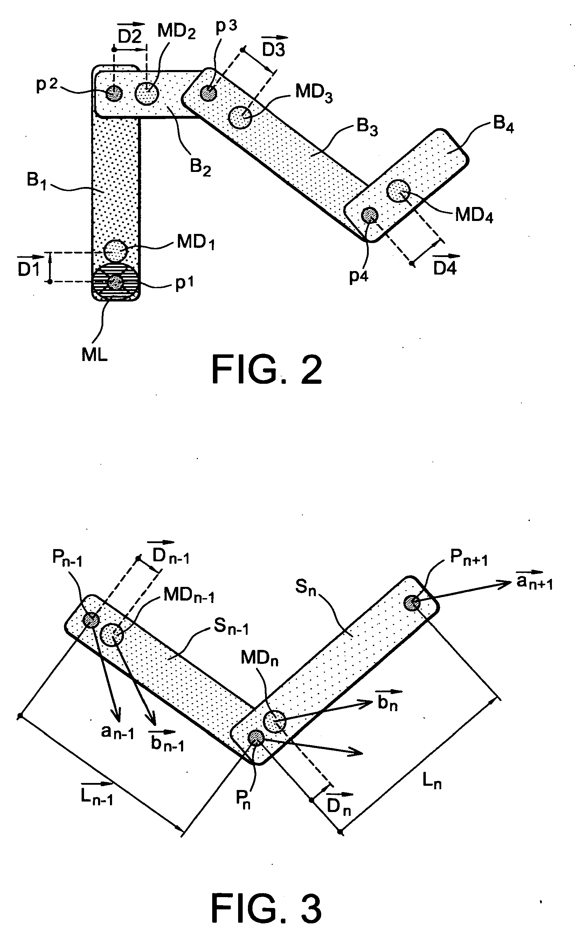

La figure 2 représente une structure articulée munie d'un dispositif de capture de mouvement selon l'invention. La structure est, par exemple, un bras de robot constitué de quatre segments articulés $B_1$, $B_2$, $B_3$, $B_4$ allant de l'épaule jusqu'à la main.

Le segment $B_1$ est muni d'un système de mesures locales ML et d'un dispositif élémentaire de mesures d'orientation $MD_1$. Le dispositif élémentaire de mesures d'orientation $MD_1$ est distant du système de mesures locales ML. Le point de fixation du système de mesures locales ML et le point de fixation du dispositif élémentaire de mesures d'orientation $MD_1$ définissent un vecteur $\vec{D_1}$ de module $D_1$ et orienté de ML vers MD1. Comme cela a été mentionné précédemment, lorsqu'un point du segment de rang 1 est fixe, le système de mesure locale ML est inutile puisqu'il est alors connu que l'accélération de ce point est nulle dans le repère de référence.

Chaque segment $B_n$ (n = 2, 3, 4) est muni d'un point d'articulation $p_n$ où s'articule le segment voisin $B_{n-1}$. Un dispositif élémentaire de mesures d'orientation $MD_n$ est placé sur chaque segment $B_n$. Le point de fixation du dispositif élémentaire de mesures d'orientation $MD_n$ est distant du point d'articulation $p_n$, le point de fixation du dispositif élémentaire de mesures d'orientation $MD_n$ et le point d'articulation $p_n$ définissant un vecteur $\vec{D_n}$ de module $D_n$ et orienté de $p_n$ vers $MD_n$.

Le dispositif de reproduction de mouvement de l'invention a pour fonction d'estimer, de proche en proche, à partir de la connaissance de l'accélération et de l'orientation du premier segment $B_1$, l'accélération des points d'articulation successifs des différents segments ainsi que les angles que font les différents segments entre eux.

Dans les schémas et discussions ci-dessous, n est l'indice générique, ou rang, d'un segment, k est un indice générique d'incrémentation du temps, $a_n$ est l'accélération du point d'articulation $p_n$ du segment de rang n dans un repère fixe de référence et $\theta_n$ est l'orientation en trois dimensions (orientation 3D) du segment de rang n dans le repère fixe. Pour des raisons de commodité, les accélérations $a_n$ et orientations $\theta_n$ sont le plus souvent notées sous forme scalaire dans la demande de brevet. Il faut cependant noter que toutes ces grandeurs sont des vecteurs de dimension trois dans le repère de référence.

La figure 3 représente une vue de détail d'une structure en mouvement équipée d'un dispositif de capture de mouvement de l'invention. Un segment $S_n$ est articulé avec un segment $S_{n-1}$ en un point d'articulation $p_n$. La longueur du segment $S_n$ est assimilée à la distance $L_n$ qui sépare le point d'articulation $p_{n+1}$ du point d'articulation $p_n$. De même, la longueur du segment $S_{n-1}$ est assimilée à la distance $L_{n-1}$ qui sépare le point d'articulation $p_n$ du point d'articulation $p_{n-1}$. Les points d'articulation $p_{n-1}$ et $p_n$ définissent un vecteur $\vec{L_n}$ orienté de $p_{n-1}$ vers $p_n$ et dont le module est égal à la distance qui sépare les points d'articulation $p_n$ et $p_{n-1}$. Le point d'articulation $p_n$ a une accélération $a_n$ et le point d'articulation $p_{n-1}$ a une accélération $a_{n-1}$. Les points de mesure des dispositifs $MD_n$ et $MD_{n-1}$ sur les segments respectifs de rang n et n-1 ont les accélération respectives $b_n$ et $b_{n-1}$.

Dans la suite de la description, l'invention sera présentée, d'une part, dans le cas particulier où les vecteurs $\vec{D_n}$ sont négligeables (les vecteurs $\vec{D_n}$ sont alors considérés comme des vecteurs nuls) et, d'autre part, dans le cas général où les vecteurs $\vec{D_n}$ ne sont pas considérés comme négligeables.

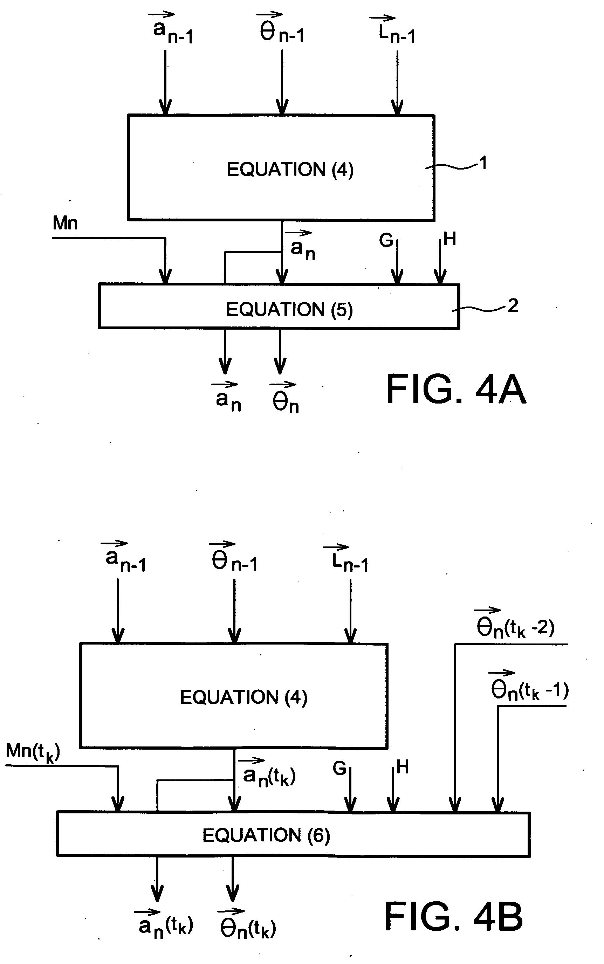

La figure 4A représente le principe général de détermination des grandeurs $\vec{a_n}$ et $\vec{\Theta_n}$ selon l'invention dans le cas particulier où les vecteurs $\vec{D_n}$ sont nuls. L'accélération $\vec{a_n}$ du point d'articulation $p_n$ est calculée à partir de l'accélération $\vec{a_{n-1}}$ du point d'articulation $p_{n-1}$, du vecteur $\vec{L_{n-1}}$ qui représente le segment de rang n-1 et du vecteur $\vec{\Theta_{n-1}}$ qui représente le vecteur orientation 3D du segment de rang n-1. Il vient, conformément à la loi de composition des mouvements :

$$\vec{a}_n = \vec{a}_{n-1} + \left(\frac{d\vec{\omega}_{n-1}}{dt}\right)\Lambda \overrightarrow{L_{n-1}} + \varpi_{n-1}\Lambda\left(\vec{\omega}_{n-1}\Lambda\overrightarrow{L_{n-1}}\right) \qquad (4)$$

dans laquelle :

- le symbole « $\Lambda$ » représente l'opérateur « produit vectoriel », et
- $\vec{\omega}_{n-1}$ = d($\vec{\Theta}_{n-1}$/dt

L'accélération $\vec{a_n}$ étant une grandeur connue, il est alors possible de calculer l'orientation $\vec{\Theta_n}$ sur la base de l'équation (5) :

7

$$\vec{\Theta}_n \;=\; F^{-1}\;(M_n,\;\vec{a}_n,\;G,\;H)\qquad(5)$$

dans laquelle :

- $M_n$ représente les mesures délivrées par le dispositif élémentaire de mesures $MD_n$ placé sur le segment de rang n, et
- G et H sont respectivement le champ de gravitation et le champ magnétique mesurés dans le repère de référence, au niveau du segment de rang n.

L'équation (5) est une équation connue en soi qui correspond à l'équation (2) rappelée ci-dessus.

La figure 4B représente, dans le cas général, les étapes essentielles du procédé de traitement de mesures mis en oeuvre dans le cadre de l'invention. Dans le cas général, l'équation qui relie l'accélération $\vec{b}_n$ du point de mesure du dispositif $MD_n$ et $\vec{\Theta}_n$ s'écrit :

$$\vec{b}_n = \vec{a}_{n-1} + \left(\frac{d\vec{\omega}_{n-1}}{dt}\right)\wedge \overrightarrow{L_{n-1}} + \vec{\omega}_{n-1}\wedge\left(\vec{\omega}_{n-1}\wedge\overrightarrow{L_{n-1}}\right) + \left(\frac{d\vec{\omega}_n}{dt}\right)\wedge \vec{D}_n + \vec{\omega}_n\wedge\left(\vec{\omega}_n\wedge\vec{D}_n\right)$$

ou encore :

$$\vec{b}_n \;=\; \vec{a}_n \;+\; \left(\frac{d\vec{\omega}_n}{dt}\right)\wedge\vec{D}_n + \vec{\omega}_n\wedge\left(\vec{\omega}_n\wedge\vec{D}_n\right)$$

Il est alors possible d'écrire la grandeur $b_n(t_k)$ sous la forme suivante :

$$b_n(t_k) \;=\; K(\vec{a}_n(t_k),\,\vec{\Theta}_n(t_{i<k}),\,\vec{\Theta}_n(t_k))$$

Le vecteur $\vec{a}_n$ est alors calculé à l'aide de l'équation (4) comme il l'était précédemment, dans le cas particulier décrit ci-dessus. Ensuite, les vecteurs $\vec{b}_n$ et $\vec{\Theta}_n$ sont calculés, à un instant $t_k$, à l'aide de l'équation (6) telle que :

$$\vec{\Theta}_n(t_k) \;=\; L^{-1}\;(M_n(t_k),\;\vec{a}(t_k),\;G,\;H,\;\vec{\Theta}_n(t_{i<k}))\quad(6)$$

où la fonction L est une fonction qui combine les fonction F et K de telle sorte que :

$$M_n(t_k) = F(\vec{b}_n(t_k),G,H,\vec{\Theta}_n(t_k))$$
$$= F[K(\vec{a}_n(t_k),\vec{\Theta}_n(t_{i<k}),\vec{\Theta}_n(t_k)),G,H,\vec{\Theta}_n(t_k)]$$
$$= L(\vec{a}_n(t_k),G,H,\vec{\Theta}_n(t_k),\vec{\Theta}_n(t_{i<k}))$$

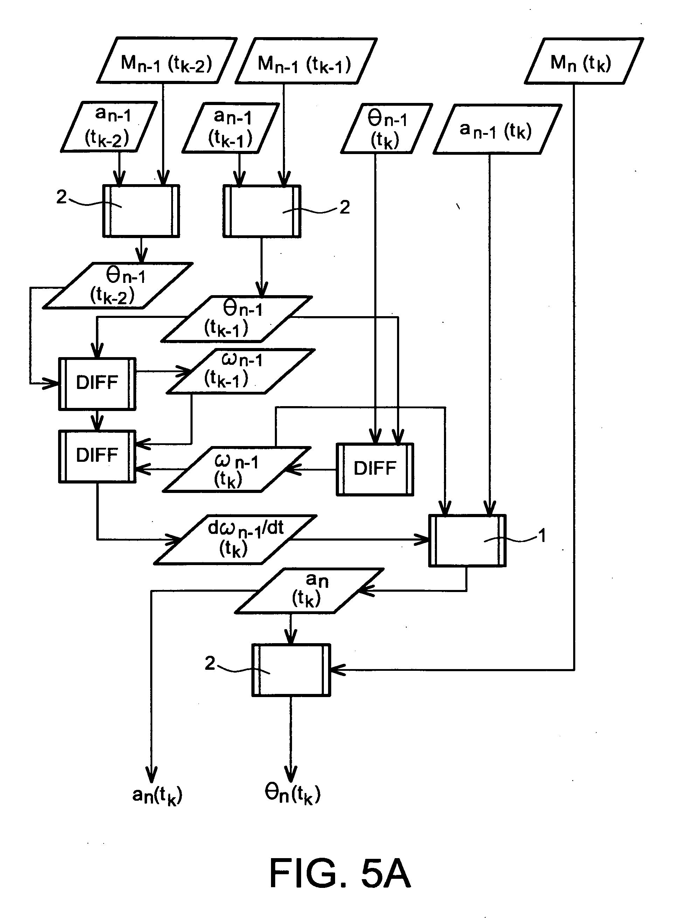

La figure 5A représente un organigramme détaillé d'une étape essentielle du procédé de traitement de mesures représenté en figure 4A.

Le bloc de traitement représenté en figure 5A détaille le calcul des grandeurs $a_n(t_k)$ et $\theta_n(t_k)$ qui sont associées, à un instant $t_k$, au segment de rang n. Les grandeurs $a_n(t_k)$ et $\theta_n(t_k)$ du segment de rang n sont déterminées à partir des données mesurées ou calculées suivantes :

- les accélérations $a_{n-1}(t_k)$, $a_{n-1}(t_{k-1})$ et $a_{n-1}(t_{k-2})$ relatives au segment de rang n-1, calculées pour trois instants diffé-

rents $t_k$, $t_{k-1}$, $t_{k-2}$, et

- les mesures $M_{n-1}(t_{k-1})$ et $M_{n-1}(t_{k-2})$ délivrées, par le dispositif élémentaire de mesures $MD_{n-1}$, aux deux instants différents $t_{k-1}$ et $t_{k-2}$,
- l'orientation $\theta_{n-1}(t_k)$ du segment de rang n-1 calculée à l'instant $t_k$, et
- les mesures $M_n(t_k)$ délivrées par le dispositif élémentaire de mesures $MD_n$ à l'instant $t_k$.

Les grandeurs $a_{n-1}(t_{k-2})$ et $M_{n-1}(t_{k-2})$ sont appliquées à un opérateur 2 qui met en oeuvre l'équation (5) et délivre l'orientation $\theta_{n-1}(t_{k-2})$. De même, les grandeurs $a_{n-1}(t_{k-1})$ et $M_{n-1}(t_{k-1})$ sont appliquées à un opérateur 2 qui met en oeuvre l'équation (5) et délivre l'orientation $\theta_{n-1}(t_{k-1})$.

Les grandeurs $\theta_{n-1}(t_{k-2})$ et $\theta_{n-1}(t_{k-1})$ et l'information d'intervalle de temps $\Delta t_{21}$ telle que :

$$\Delta t_{21} = t_{k-2} - t_{k-1}$$

sont ensuite appliquées à un opérateur différentiateur DIFF qui calcule la grandeur $\omega_{n-1}(t_{k-1})$ telle que :

$$\omega_{n-1}(t_{k-1}) = (\theta_{n-1}(t_{k-2}) - \theta_{n-1}(t_{k-1})) / \Delta t_{21}.$$

Les grandeurs $\omega_{n-1}(t_k)$ et d $(\omega_{n-1}(t_k))/dt$ sont ensuite calculées :

- la grandeur $\omega_{n-1}(t_k)$ est calculée à l'aide d'un opérateur différentiateur DIFF de sorte que : $\omega_{n-1}(t_k) = (\theta_{n-1}(t_{k-1}) - \theta_{n-1}(t_k)) / \Delta t_{10}$, où $\theta_{n-1}(t_{k-1})$ est la grandeur calculée ci-dessus, $\theta_{n-1}(t_k)$ est connu (calculé précédemment), et $\Delta t_{10} = t_{k-1} - t_k$, et
- la grandeur $d(\omega_{n-1}(t_k))/dt$ est calculée à l'aide d'un opérateur différentiateur DIFF de sorte que : $d(\omega_{n-1}(t_k)) / dt = (\omega_{n-1}(t_{k-1}) - \omega_{n-1}(t_k)) / \Delta t_{10}$ où $\omega_{n-1}(t_{k-1})$ et $\omega_{n-1}(t_k)$ sont les grandeurs calculées ci-dessus, et $\Delta t_{10} = t_{k-1} - t_k$.

Les grandeurs $a_{n-1}(t_k)$, $\omega_{n-1}(t_k)$ et $d(\omega_{n-1}(t_k))/dt$ sont alors appliquées à un opérateur 1 qui met en oeuvre l'équation (4) et délivre la grandeur $a_n(t_k)$. La grandeur calculée $a_n(t_k)$ et la mesure connue prélevée $M_n(t_k)$ sont ensuite appliquées à un opérateur 2 qui met en oeuvre l'équation (5) et délivre la grandeur d'orientation $\theta_n(t_k)$.

Le traitement des mesures acquises par le dispositif de capture de mouvement articulé de l'invention conduit à la détermination, pour chaque segment de la structure en mouvement, de son accélération au point d'articulation et de son orientation dans un repère de référence. Il est alors possible de décrire le mouvement de la structure, par exemple sur un écran.

Comme cela apparaît clairement en référence à la figure 5A, la détermination du couple [$a_n(t_k)$, $\theta_n(t_k)$] d'un segment de rang n à l'instant $t_k$ est déduite, entre autres, d'informations relatives au segment de rang n-1 aux instants antérieurs $t_{k-1}$ et $t_{k-2}$. En conséquence, il est clair que l'ensemble des données d'accélération et d'orientation relatives à l'ensemble des segments de la structure ne peut pas être connu dès la première mesure. Il est ainsi nécessaire d'acquérir un certain nombre de mesures avant que le mouvement articulé ne puisse être reproduit dans sa totalité.

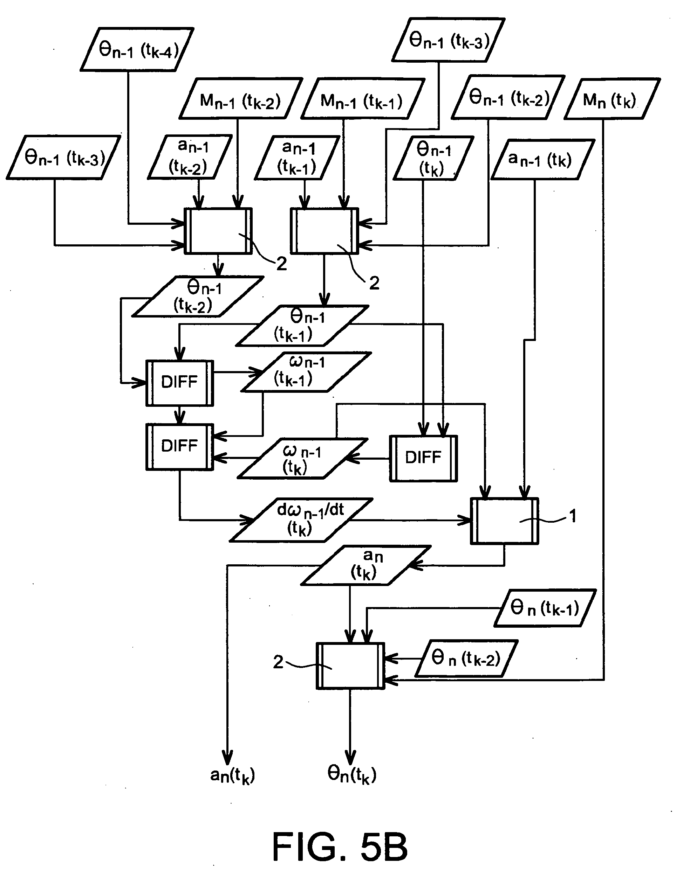

La figure 5B représente un organigramme détaillé d'une étape essentielle du procédé de traitement de mesures représenté en figure 4B.

En plus des données mentionnées en référence à la figure 5A, les grandeurs $\vec{a}_n(t_k)$ et $\vec{\Theta}_n(t_k)$ relatives au segment de rang n sont ici également déterminées à partir des orientations $\theta_n(t_{k-1})$ et $\theta_n(t_{k-2})$ calculées, pour le segment n, aux instants $t_{k-1}$ et $t_{k-2}$.

Les grandeurs $a_{n-1}(t_{k-2})$, $\theta_{n-1}(t_{k-3})$, $\theta_{n-1}(t_{k-4})$ et $M_{n-1}(t_{k-2})$ sont alors appliquées à un opérateur 2 qui met en oeuvre l'équation (6) et délivre l'orientation $\theta_{n-1}(t_{k-2})$. De même, les grandeurs $a_{n-1}(t_{k-1})$, $\theta_{n-1}(t_{k-2})$, $\theta_{n-1}(t_{k-3})$ et $M_{n-1}(t_{k-1})$ sont appliquées à un opérateur 2 qui met en oeuvre l'équation (6) et délivre l'orientation $\theta_{n-1}(t_{k-1})$.

Les grandeurs $a_{n-1}(t_k)$, $\omega_{n-1}(t_k)$ et $d(\omega_{n-1}(t_k))/dt$ sont alors appliquées à un opérateur 1 qui met en oeuvre l'équation (4) et délivre la grandeur $a_n(t_k)$. Les orientations $\theta_n(t_{k-1})$ et $\theta n(t_{k-2})$ estimées aux deux instants précédents l'instant $t_k$, la grandeur calculée $a_n(t_k)$ et la mesure connue prélevée $M_n(t_k)$ sont ensuite appliquées à un opérateur 2 qui met en oeuvre l'équation (6), où $\omega_n(t_k)$ et $d\omega(t_k)$ sont données comme précédemment par l'opérateur DIFF :

$$\omega_n(t_k) = (\theta_n(t_{k-1}) - \theta_n(t_k)) / \Delta t10$$

$$d(W_n(t_k))/dt = (W_n(t_{k-1}) - W(t_k))/\Delta t10$$

où

$$W_n(t_{k-1}) = (\theta_n(t_{k-2}) - \theta_n(t_{k-1}))/\Delta t21$$

L'opérateur 2 délivre alors la grandeur d'orientation $\theta_n(t_k)$.

Le traitement des mesures acquises par le dispositif de capture de mouvement articulé de l'invention conduit à la détermination, pour chaque segment de la structure en mouvement, de son accélération au point d'articulation et de son orientation dans un repère de référence. Il est alors possible de décrire le mouvement de la structure, par exemple sur un écran.

Comme cela apparaît clairement, par exemple en référence à la figure 5A, la détermination du couple [$a_n(t_k)$, $\theta_n(t_k)$] d'un segment de rang n à l'instant $t_k$ est déduite, entre autres, d'informations relatives au segment de rang n-1 aux instants antérieurs $t_{k-1}$ et $t_{k-2}$. En conséquence, il est clair que l'ensemble des données d'accélération et d'orientation relatives à l'ensemble des segments de la structure ne peut pas être connu dès la première mesure. Il est ainsi nécessaire d'acquérir un certain nombre de mesures avant que le mouvement articulé ne puisse être reproduit dans sa totalité.

De même, dans le cas général où les vecteurs $\vec{D}_n$ ne sont pas considérés comme nuls, il faut connaître deux orientations successives précédentes du segment de rang n pour initialiser le procédé. Ces orientations peuvent être obtenues par exemple lorsque le segment est immobile en utilisant le procédé décrit dans la demande de brevet FR 2838185. Par contre, comme cela a été montré ci-dessus, dans le cas où les moyens de mesures représentatifs de l'orientation du segment de rang n sont suffisamment proches du point d'articulation pn, il n'est pas nécessaire de connaître les deux orientations successives précédentes et le procédé est simplifié.

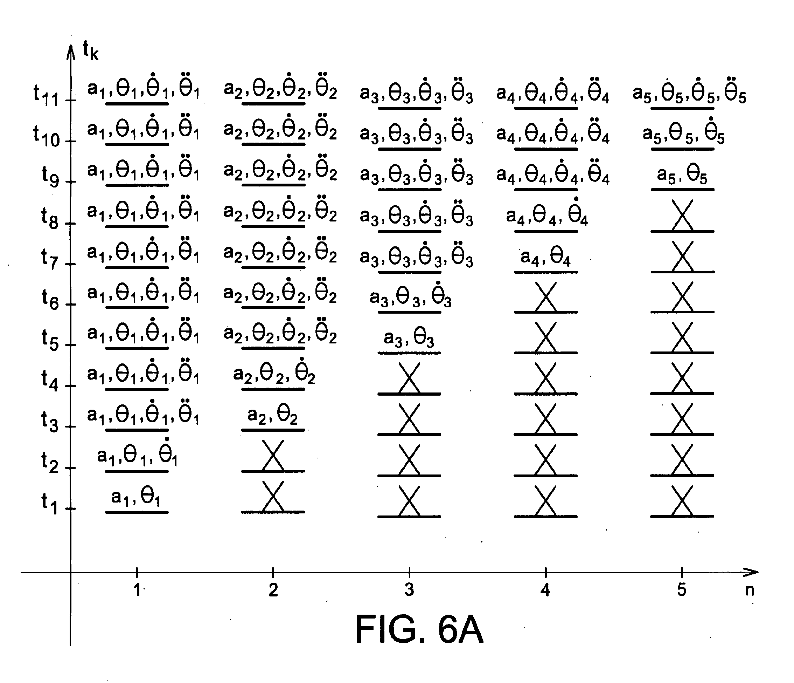

La figure 6A illustre, de façon symbolique, dans le cas particulier où les vecteurs $\vec{D}_n$ sont considérés comme nuls, l'évolution au cours du temps des données d'accélération et d'orientation obtenues pour les différents segments d'une structure à cinq segments articulés

Sur la figure 6A, l'axe horizontal représente le rang n des segments qui composent la structure et l'axe vertical représente des instants de mesure successifs $t_k$. A l'intersection d'un rang n et d'un instant $t_k$ sont indiquées les grandeurs (accélération et orientation) qui sont connues à l'instant $t_k$, pour le segment de rang n. Ces grandeurs sont constituées de données de mesure et/ou de données déduites de données de mesure.

Afin de ne pas alourdir la figure 6A, la grandeur $d\theta_n/dt$ est représentée par le symbole $\dot{\theta}_n$ et la grandeur $d^2\theta_n/dt^2$ est représentée par le symbole $\ddot{\theta}_n$. Par ailleurs, il vient :

- $d\theta_n(t_k) = \theta_n(t_k) - \theta_n(t_{k-1})$,
- $dt(t_k) = t_k - t_{k-1}$,
- $d^2\theta_n(t_k) = d\theta_n(t_k) - d\theta_n(t_{k-1})$,
- $dt^2(t_k) = t_k - t_{k-1}$.

A l'instant $t_1$, les seules grandeurs connues relatives aux segments sont les suivantes :

$$a_1(t_1), \quad \theta_1(t_1).$$

Ces données sont bien sûr insuffisantes pour décrire le mouvement de la structure.

A l'instant $t_2$, les grandeurs connues relatives aux segments de rangs 1 à 5 sont les suivantes :

$$a_1(t_2), \quad \theta_1(t_2), \quad d\theta_1/dt(t_2).$$

Ces données sont toujours insuffisantes pour décrire le mouvement de la structure.

A l'instant $t_3$, les grandeurs connues relatives aux segments sont les suivantes :

- $a_1(t_3)$, $\theta_1(t_3)$, $d\theta_1/dt(t_3)$, $d^2\theta_1/dt^2(t_3)$,
- $a_2(t_3)$, $\theta_2(t_3)$.

Ces données sont toujours insuffisantes pour décrire le mouvement de la structure.
A l'instant $t_4$, les grandeurs connues sont les suivantes :

- $a_1(t_4)$, $\theta_1(t_4)$, $d\theta_1/dt(t_4)$, $d^2\theta_1/dt^2(t_4)$,
- $a_2(t_4)$, $\theta_2(t_4)$, $d\theta_2/dt(t_4)$,

Ces données sont toujours insuffisantes pour décrire le mouvement de la structure.
A l'instant $t_5$, les grandeurs connues sont les suivantes :

- $a_1(t_5)$, $\theta_1(t_5)$, $d\theta_1/dt(t_5)$, $d^2\theta_1/dt^2(t_5)$,
- $a_2(t_5)$, $\theta_2(t_5)$, $d\theta_2/dt(t_5)$, $d^2\theta_2/dt^2(t_5)$,
- $a_3(t_5)$, $\theta_3(t_5)$.

Ces données sont toujours insuffisantes pour décrire le mouvement de la structure.
A l'instant $t_6$, les grandeurs connues relatives aux segments de rangs 1 à 5 sont respectivement les suivantes :

- $a_1(t_6)$, $\theta_1(t_6)$, $d\theta_1/dt(t_6)$, $d^2\theta_1/dt^2(t_6)$,
- $a_2(t_6)$, $\theta_2(t_6)$, $d\theta_2/dt(t_6)$, $d^2\theta_2/dt^2(t_6)$,
- $a_3(t_6)$, $\theta_3(t_6)$, $d\theta_3/dt(t_6)$.

Ces données sont toujours insuffisantes pour décrire le mouvement de la structure.
A l'instant $t_7$, les grandeurs connues relatives aux segments de rangs 1 à 5 sont respectivement les suivantes :

- $a_1(t_7)$, $\theta_1(t_7)$, $d\theta_1/dt(t_7)$, $d^2\theta_d dt^2(t_7)$,
- $a_2(t_7)$, $\theta_2(t_7)$, $d\theta_2/dt(t_7)$, $d^2\theta_2/dt^2(t_7)$,
- $a_3(t_7)$, $\theta3(t_7)$, $d\theta_3/dt(t_7)$, $d^2\theta_3/dt^2(t7)$,
- $a_4(t_7)$, $\theta_4(t_7)$.

Ces données sont toujours insuffisantes pour décrire le mouvement de la structure.
A l'instant $t_8$, les grandeurs connues sont les suivantes :

- $a_1(t_8)$, $\theta_1(t_8)$ , $d\theta_1/dt(t_8)$, $d^2\theta_1/dt^2(t_8)$,
- $a_2(t_8)$, $\theta_2(t_8)$, $d\theta_2/dt(t_8)$, $d^2\theta_2/dt^2(t_8)$,
- $a_3(t_8)$, $\theta3(t_8)$, $d\theta_3/dt(t_8)$, $d^2\theta_3/dt^2(t_8)$,
- $a_4(t_8)$, $\theta_4(t_8)$, $d\theta_4/dt(t_8)$.

A l'instant $t_9$, les grandeurs connues sont les suivante :

- $a_1(t_9)$, $\theta1(t_9)$, $d\theta_1/dt(t_9)$, $d^2\theta_1 dt^2(t_9)$,
- $a_2(t_9)$, $\theta_2(t_9)$, $d\theta_2/dt(t_9)$, $d^2\theta_2/dt^2(t_9)$,
- $a_3(t_9)$, $\theta3(t_9)$, $d\theta_3/dt(t_9)$, $d^2\theta_3/dt^2(t_9)$,
- $a_4(t_9)$, $\theta_4(t_9)$, $d\theta_4/dt(t_9)$, $d^2\theta_4/dt^2(t_9)$.
- $a_5(t_9)$, $\theta_5(t_9)$.

Ces données permettent maintenant de décrire complètement le mouvement de la structure. Si l'on continue la représentation pour les instants ultérieurs $t_{10}$ et $t_{11}$, il vient :

- à l'instant $t_{10}$, les grandeurs connues relatives aux segments de rangs 1 à 5 sont respectivement les suivantes :

    - $a_1(t_{10})$, $\theta_1(t_{10})$, $d\theta_1/dt(t_{10})$, $d^2\theta_1/dt^2(t10)$,
    - $a_2(t_{10})$, $\theta_2(t_{10})$, $d\theta_2/dt(t_{10})$, $d^2\theta_2/dt^2(t_{10})$,
    - $a_3(t_{10})$, $\theta3(t_{10})$, $d\theta_3/dt(t_{10})$, $d^2\theta_3/dt^2(t_{10})$
    - $a_4(t_{10})$, $\theta_4(t_{10})$, $d\theta_4/dt(t_{10})$, $d^2\theta_4/dt^2(t_{10})$,
    - $a_5(t_{10})$, $\theta_5(t_{10})$, $d\theta_5/dt(t_{10})$ ; et

- à l'instant $t_{11}$, les grandeurs connues relatives aux segments de rangs 1 à 5 sont respectivement les suivantes :

    - $a_1(t_{11})$, $\theta_1(t_{11})$, $d\theta_1/dt(t_{11})$, $d^2\theta_1/dt^2(t_{11})$.

- $a_2(t_{11})$, $\theta_2(t_{11})$, $d\theta_2/dt(t_{11})$, $d^2\theta_2/dt^2(t_{11})$,
- $a_3(t_{11})$, $\theta3(t_{11})$, $d\theta_3/dt(t_{11})$, $d^2\theta_3/dt^2(t_{11})$,
- $a_4(t_{11})$, $\theta_4(t_{11})$, $d\theta_4/dt(t_{11})$, $d^2\theta_4/dt^2(t_{11})$,
- $a_5(t_{11})$, $\theta_5(t_{11})$, $d\theta_5/dt(t_{11})$, $d^2\theta_5/dt^2(t_{11})$,

Le mouvement articulé de la structure à cinq segments est totalement défini dès lors que les accélérations et les orientations des cinq segments (n=5) sont connues, c'est-à-dire à partir de l'instant $t_9$ (k=9). De même, on constate, par exemple, que pour une structure à trois segments (n=3), les accélérations et les orientations des trois segments sont connues à partir de l'instant $t_5$ (k=5).

Il est ainsi possible d'établir, entre le nombre entier n et le nombre entier k, une relation qui traduit le fait que le dispositif de capture de mouvement fonctionne correctement, c'est-à-dire délivre toutes les informations d'accélération et d'orientation nécessaires pour tous les segments de la structure. Cette relation s'écrit :

$$k > 2n - 2.$$

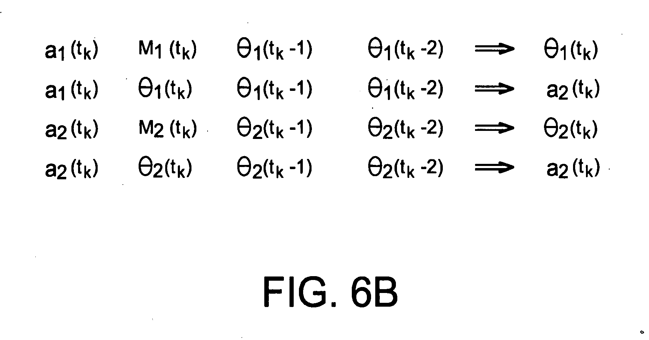

La figure 6B illustre, dans le cas général, les résultats de calculs des données d'accélération et d'orientation obtenues, de proche en proche, pour les différents segments d'une structure à segments articulés. Le calcul des données d'accélération et d'orientation est décrit ci-dessous pour les trois premiers segments.

Cas du premier segment

Dans une première étape, on utilise les mesures $a_1(t_k)$ et $M_1(t_k)$ qui correspondent respectivement à l'accélération mesurée (ou calculée) sur le segment 1 (grâce aux premiers moyens de mesure ML) et aux mesures délivrées par les seconds moyens de mesure (MD1). On utilise également les orientations $\theta_1(t_{k-1})$ et $\theta_1(t_{k-2})$ du premier segment données (ou calculées) pour les instants précédents ($t_{k-1}$ et $t_{k-2}$).

Grâce à ces quatre informations on peut calculer l'orientation du segment 1 à l'instant tk . $\theta_1(t_k)$.

Dans une deuxième étape, on utilise $a_1(t_k)$ l'accélération mesurée (ou calculée) sur le segment 1 (grâce aux premiers moyens de mesure ML), ainsi que les orientations du premier segment $\theta_1(t_k)$ calculée à l'étape précédente et $\theta_1(t_{k-1})$ et $\theta_1(t_{k-2})$ celles données (ou calculées) pour les instants précédent ($t_{k-1}$ et $t_{k-2}$). On calcule avec ces grandeurs l'accélération $a_2(t_k)$ au niveau de l'articulation p2.

Cas du deuxième segment

Dans une première étape, on utilise l'accélération $a_2(t_k)$ calculée à l'étape précédente, les mesures $M_2(t_k)$ des moyens de mesures $MD_2$ du deuxième segment à l'instant $t_k$. On utilise également les orientations $\theta_2(k_{k-1})$ et $\theta_2(t_{k-2})$ du deuxième segment données (ou calculées) pour les instants précédents ($t_{k-1}$ et $t_{k-2}$).

Grâce à ces quatre informations on peut calculer l'orientation du segment 1 à l'instant $t_k$ : $\theta_1(t_k)$.

Dans une deuxième étape, on utilise $a_2(t_k)$ l'accélération calculé à la deuxième étape du premier segment, ainsi que les orientations du deuxième segment $\theta_2(t_k)$ calculée à l'étape précédente et $\theta_2(t_{k-1})$ et $\theta_2(t_{k-2})$ données (ou calculées) pour les instants précédent $t_{k-1}$ et $t_{k-2}$. On calcule avec ces grandeurs l'accélération $a_3(t_k)$ au niveau de l'articulation p3.

Cas du troisième segment

On réalise les mêmes deux étapes que pour le deuxième segment en substituant l'indice 4 à l'indice 3, l'indice 3 à l'indice 2, et l'indice 2 à l'indice 1.

Il en va ainsi de suite jusqu'au Nième segment : on réalise les mêmes deux étapes que pour le deuxième segment en substituant l'indice N+1 à l'indice 3, l'indice N à l'indice 2 et l'indice N-1 à l'indice 1.

Lorsque tous les segments ont été considérés, on attend l'instant $t_k$ suivant et on recommence.

Dans le cas général, on note que, pour connaître l'orientation estimée d'un segment à l'instant $t_k$, il est nécessaire de connaître les orientations estimées de ce même segment aux deux instants précédents $t_{k-1}$ et $t_{k-2}$. En conséquence, pour le premier instant du calcul, il est nécessaire d'initialiser les valeurs des orientations aux instants précédents. Pour cela on pourra, par exemple, faire des mesures statiques pour lesquelles les accélérations sont faibles et peuvent en conséquence être négligées; les angles pourront alors être calculés de la manière décrite dans la demande de brevet FR 2 838 185. On peut également utiliser d'autres moyens pour initialiser les angles (codeurs angulaires, mise à une position initiale contrainte, etc.).

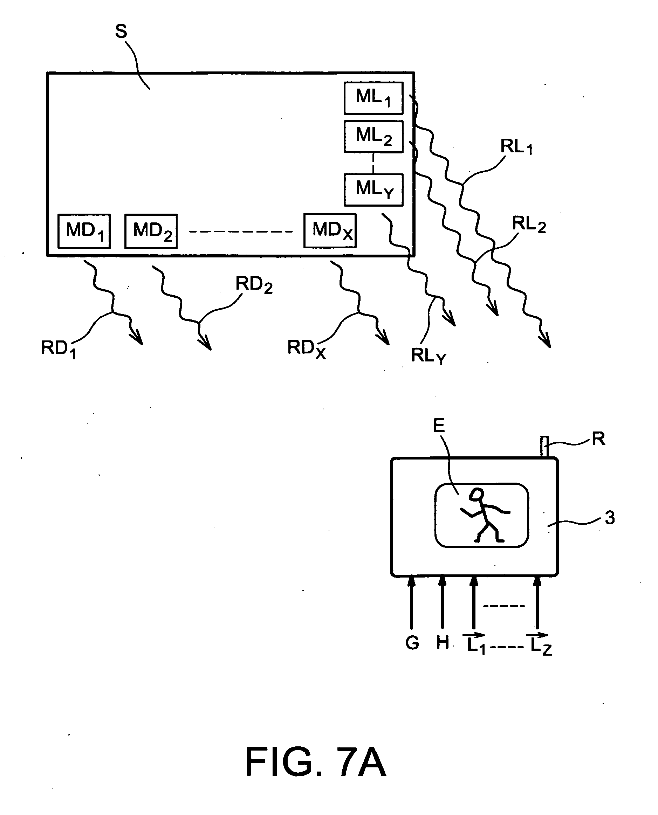

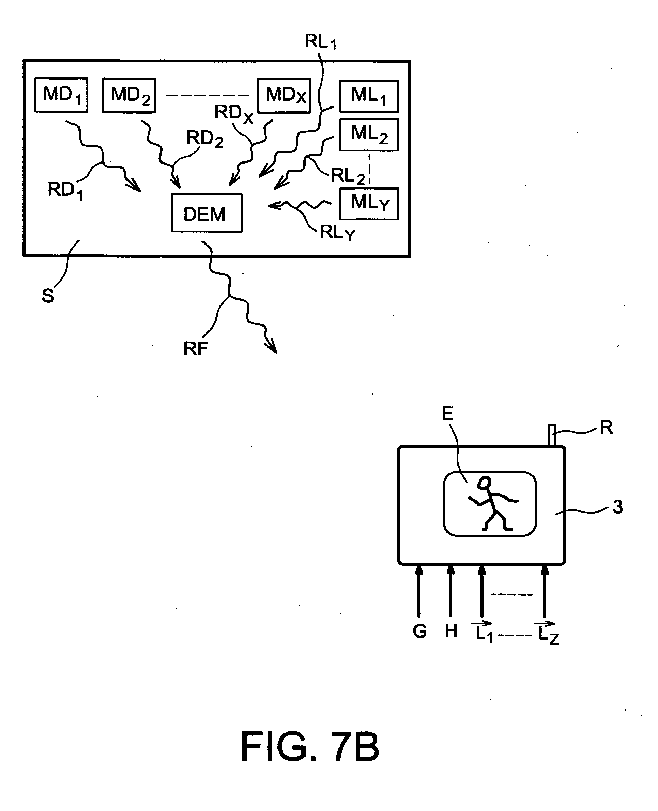

Les figures 7A et 7B représentent deux modes de réalisation d'un dispositif de reproduction de mouvement selon l'invention. La structure S constituée de n segments articulés est représentée de façon symbolique par un rectangle. La structure S, par exemple un homme ou un robot, est munie d'un ensemble de dispositifs $MD_i$ (i=11, 2, ..., n) et d'un ensemble de systèmes de mesures locales $ML_j$ (j=1, 2, ..., m) . Les dispositifs $MD_i$ et les systèmes $ML_j$ sont distribués sur la structure comme cela a été décrit précédemment. Comme décrit précédemment également, bien que m systèmes de mesures locales soient représentés sur les figures 7A et 7B, un seul système de mesures locales suffit pour mettre en oeuvre l'invention.

Dans le premier mode de réalisation (figure 7A), les mesures délivrées par les dispositifs $MD_i$ et les mesures délivrées par les systèmes de mesures locales $ML_j$ sont transmises, par les signaux radioélectriques respectifs $RD_i$ et $RL_j$, vers un système de calcul 3, par exemple un ordinateur. Le dispositif de reproduction de mouvement comprend alors des moyens de transmission radioélectrique. Le système de calcul 3 est muni d'une antenne de réception R qui reçoit les signaux $RD_i$ et $RL_j$. Le système de calcul 3 reçoit, par ailleurs, comme paramètres d'entrée, la valeur G du champ de gravitation local dans le repère de référence, la valeur H du champ magnétique local dans le repère de référence, et les coordonnées des différents vecteurs $\vec{L}_i$ (i=1, 2, ..., n) qui représentent les différents segments.

Le système de calcul 3 met alors en oeuvre un traitement des données conforme à ce qui a été décrit ci-dessus en référence aux figures 5 et 6. Un dispositif d'affichage E, par exemple un écran, permet alors de visualiser le mouvement de la structure articulée.

La figure 7B diffère de la figure 7A en ce que les signaux radioélectriques $RD_i$ et $RL_j$ ne sont pas ici directement transmis au système de calcul 3 mais sont transmis à une unité intermédiaire DEM fixée sur la structure S. L'unité DEM transmet alors les données qu'elle reçoit sous la forme d'un signal radioélectrique RF au système de calcul 3.

La présence d'une unité intermédiaire DEM sur la structure S permet avantageusement de mettre en oeuvre un autre mode de réalisation de l'invention. En effet, dans le cas où la structure S se meut à une grande distance du système de calcul 3, il est possible que la portée du signal RF se détériore. Une carte mémoire placée dans l'unité intermédiaire DEM peut alors enregistrer les signaux $RD_i$ et $RL_j$. Le traitement des données peut alors être effectué postérieurement à la capture des mesures, une fois le mouvement exécuté, à partir de la lecture des données enregistrées sur la carte mémoire.

**Revendications**

1. Dispositif de capture de mouvement d'une structure constituée de N segments solides successifs articulés les uns par rapport aux autres à partir d'un segment de rang 1 jusqu'à un segment de rang N, N étant un nombre entier supérieur ou égal à 2, le segment de rang n (n = 2, ..., N) étant articulé avec le segment de rang n-1 au niveau d'un point d'articulation $p_n$, **caractérisé en ce qu'**il comprend :

   - des premiers moyens (ML) qui délivrent une information apte à restituer un vecteur d'accélération absolue $\vec{a}_1$ d'un point du segment de rang 1 dans un repère de référence, à des instants successifs $t_k$, k étant un nombre entier supérieur ou égal à 1,
   - des seconds moyens de mesure ($MD_1$) fixés sur le segment de rang 1 et qui délivrent, à chaque instant $t_k$, une mesure ($M_1$) représentative d'un vecteur orientation $\vec{\Theta}_1$ du segment de rang 1 dans le repère de référence, et
   - des moyens de mesure supplémentaires ($MD_n$) fixés sur chaque segment de rang n (n = 2, ..., N) et qui délivrent, à chaque instant $t_k$, une mesure représentative d'un vecteur orientation $\vec{\Theta}_n$ du segment de rang n.

2. Dispositif de capture de mouvement selon la revendication 1, dans lequel les seconds moyens de mesure ($MD_1$) et les moyens de mesure supplémentaires ($MD_n$) sont constitués d'un accéléromètre et d'un capteur qui délivre une mesure d'un champ physique uniforme présent dans l'espace où se meut la structure et de direction connue dans le repère de référence.

3. Dispositif de capture de mouvement selon la revendication 2, dans lequel les seconds moyens de mesure ($MD_1$) et les moyens de mesure supplémentaires ($MD_n$) comprennent, en outre, au moins un axe gyrométrique.

4. Dispositif de capture de mouvement selon l'une quelconque des revendication 2 ou 3, dans lequel le capteur qui délivre une mesure d'un champ physique uniforme de direction connue dans le repère de référence est un magnétomètre.

5. Dispositif de capture de mouvement selon l'une quelconque des revendications 2 ou 3, dans lequel le capteur qui délivre une mesure d'un champ physique uniforme de direction connue dans le repère de référence est une cellule photoélectrique.

**6.** Dispositif selon l'une quelconque des revendications précédentes, dans lequel les premiers moyens (ML) sont des moyens de mesure constitués d'un mesureur de vitesse de sorte que la donnée apte à restituer un vecteur d'accélération absolue du segment de rang 1 est la vitesse du point.

**7.** Dispositif selon l'une quelconque des revendications 1 à 5, dans lequel les premiers moyens (ML) sont des moyens de mesure constitués d'un mesureur de position de sorte que la donnée apte à restituer un vecteur d'accélération absolue d'un point du segment de rang 1 est la position du point.

**8.** Dispositif de reproduction de mouvement d'une structure constituée de N segments solides successifs articulés les uns par rapport aux autres à partir d'un segment de rang 1 jusqu'à un segment de rang N, N étant un nombre entier supérieur ou égal à 2, le segment de rang n (n = 2, ..., N) étant articulé avec le segment de rang n-1 au niveau d'un point d'articulation $p_n$, **caractérisé en ce qu'**il comprend :

  - un dispositif de capture de mouvement selon l'une quelconque des revendications 1 à 7 dans lequel les moyens de mesure supplémentaires ($MD_n$) d'un segment de rang n sont positionnés à proximité du point d'articulation $p_n$ de telle sorte que la distance qui sépare les moyens de mesure supplémentaire ($MD_n$) d'un segment de rang n du point d'articulation $p_n$ est considérée comme nulle, et
  - des moyens de calcul (3) qui calculent, à chaque instant $t_k$ :

    a) le vecteur d'accélération absolue $\vec{a}_1$ dans le repère de référence, à partir de l'information délivrée par les premiers moyens,
    b) le vecteur d'orientation $\vec{\Theta}_1$ du segment de rang 1 dans le repère de référence, à partir du vecteur d'accélération absolue $\vec{a}_1$ et de la mesure représentative ($M_1$) du vecteur orientation ($\vec{\Theta}_1$) du segment de rang 1 ;
    c) un vecteur d'accélération $\vec{a}_n$ (n ≥ 2) du point d'articulation $p_n$ dans le repère de référence, à partir de l'équation :

$$\vec{a}_n = \vec{a}_{n-1} + \left(\frac{d\vec{\omega}_{n-1}}{dt}\right) \wedge \overrightarrow{L_{n-1}} + \vec{\omega}_{n-1} \wedge \left(\vec{\omega}_{n-1} \wedge \overrightarrow{L_{n-1}}\right)$$

    où $\vec{\omega}_n$ = d($\vec{\Theta}_n$)/dt et $\vec{L}_n$ étant un vecteur orienté du point d'articulation $p_{n-1}$ vers le point d'articulation $p_n$ et dont le module a pour valeur la distance qui sépare le point d'articulation $p_n$ du point d'articulation $p_{n-1}$; et
    d) le vecteur d'orientation $\vec{\Theta}_n$ (n ≥ 2) du segment de rang n à partir du vecteur d'accélération $\vec{a}_n$ et de la mesure représentative ($M_n$) de l'orientation du segment de rang n.

**9.** Dispositif de reproduction de mouvement d'une structure constituée de N segments solides successifs articulés les uns par rapport aux autres à partir d'un segment de rang 1 jusqu'à un segment de rang N, N étant un nombre entier supérieur ou égal à 2, le segment de rang n (n = 2, ..., N) étant articulé avec le segment de rang n-1 au niveau d'un point d'articulation $p_n$, **caractérisé en ce qu'**il comprend :

  - un dispositif de capture de mouvement selon l'une quelconque des revendications 1 à 7 dans lequel les moyens de mesure supplémentaires ($MD_n$) d'un segment de rang n sont distants du point d'articulation pn, et
  - des moyens de calcul (3) qui calculent, à chaque instant $t_k$ :

    a) le vecteur d'accélération absolue $\vec{a}_1$ dans le repère de référence, à partir de l'information délivrée par les premiers moyens,
    b) le vecteur d'orientation $\vec{\Theta}_1$, du segment de rang 1 dans le repère de référence, à partir du vecteur d'accélération absolue $\vec{a}_1$ et de la mesure représentative ($M_1$) du vecteur orientation ($\vec{\Theta}_1$) du segment de rang 1 ;
    c) un vecteur d'accélération $\vec{a}_n$ (n ≥ 2) du point d'articulation $p_n$ dans le repère de référence, à partir de l'équation :

$$\vec{a}_n = \vec{a}_{n-1} + \left(\frac{d\vec{\omega}_{n-1}}{dt}\right) \wedge \overrightarrow{L_{n-1}} + \vec{\omega}_{n-1} \wedge \left(\vec{\omega}_{n-1} \wedge \overrightarrow{L_{n-1}}\right) \cdot$$

où $\vec{\omega}_n$ = d($\vec{\Theta}_n$)/dt, $\vec{L}_n$ étant un vecteur orienté du point d'articulation $p_{n-1}$ vers le point d'articulation $p_n$ et dont le module a pour valeur la distance qui sépare le point d'articulation $p_n$ du point d'articulation $p_{n-1}$ ; et

d) le vecteur d'orientation $\vec{\Theta}_n$ (n ≥ 2) et un vecteur d'accélération $\vec{b}_n$ du point de mesure des moyens de mesure supplémentaire fixés sur le segment de rang n à partir du vecteur d'accélération $\vec{a}_n$, de la mesure représentative ($M_n$) de l'orientation du segment de rang n, et des vecteurs d'orientation du segment de rang n à au moins deux instants qui précèdent l'instant $t_k$, avec $\vec{b}_n$ tel que :

$$\vec{b}_n = \vec{a}_n + \left(\frac{d\bar{\omega}_n}{dt}\right) \wedge \vec{D}_n + \bar{\omega}_n \wedge \left(\bar{\omega}_n \wedge \vec{D}_n\right)$$

où $\vec{D}_n$ est un vecteur orienté du point d'articulation $p_n$ vers les moyens de mesure supplémentaires du segment de rang n et dont le module est sensiblement égal à la distance qui sépare le point d'articulation $p_n$ des moyens de mesure supplémentaires du segment de rang n.

**10.** Dispositif de reproduction de mouvement selon l'une quelconque des revendications 8 ou 9, dans lequel des moyens de transmission radioélectrique transmettent des signaux électriques élémentaires ($RD_n$, $RL_m$) représentatifs des mesures délivrées par les premiers moyens de mesure (ML) et les seconds moyens de mesure ($MD_n$) vers les moyens de calcul (3) .

**11.** Dispositif de reproduction de mouvement selon la revendication 10, dans lequel les moyens de transmission comprennent une unité intermédiaire (DEM) qui reçoit les signaux électriques élémentaires ($RD_1$, ..., $RD_X$, $RL_1$, ..., $RL_Y$) et qui réémet un signal électrique (RF) représentatif des signaux électriques élémentaires vers les moyens de calcul (3).

**12.** Dispositif selon l'une quelconque des revendications précédentes, dans lequel des moyens de mémorisation mémorisent les mesures délivrées par les premiers moyens de mesure (ML) et les seconds moyens de mesure ($MD_n$).

**13.** Dispositif selon la revendication 12, dans lequel les moyens de mémorisation sont placés sur la structure.

**14.** Procédé de capture de mouvement d'une structure constituée de N segments solides successifs articulés les uns par rapport aux autres à partir d'un segment de rang 1 jusqu'à un segment de rang N, N étant un nombre entier supérieur ou égal à 2, le segment de rang n (n = 2, ..., N) étant articulé avec le segment de rang n-1 au niveau d'un point d'articulation $p_n$, **caractérisé en ce qu'**il comprend :

- au moins une détermination d'une information apte à restituer un vecteur d'accélération absolue $\vec{a}_1$ d'un point du segment de rang 1 dans un repère de référence, à des instants successifs $t_k$, k étant un nombre entier supérieur ou égal à 1,
- au moins une mesure représentative d'un vecteur orientation $\vec{\Theta}_1$ du segment de rang 1 dans le repère de référence, à chacun des instant successifs $t_k$, et
- pour chaque segment de rang n, au moins une mesure supplémentaire d'un vecteur orientation $\vec{\Theta}_n$ du segment de rang n dans le repère de référence, à chacun des instant successifs $t_k$.

**15.** Procédé de capture de mouvement selon la revendication 14, dans lequel la mesure représentative du vecteur orientation $\vec{\Theta}_1$ du segment de rang 1 dans le repère de référence et la mesure représentative du vecteur orientation $\vec{\Theta}_n$ du segment de rang n sont, chacune, une mesure d'un champ uniforme présent dans l'espace où se meut la structure et de direction connue dans le repère de référence.

**16.** Procédé de capture de mouvement selon la revendication 14 ou 15, dans lequel l'information apte à restituer un vecteur d'accélération absolue $\vec{a}_1$ d'un point du segment de rang 1 dans un repère de référence est la vitesse du point dans le repère de référence.

**17.** Procédé de capture de mouvement selon la revendication 14 ou 15, dans lequel l'information apte à restituer un vecteur d'accélération absolue $\vec{a}_1$ d'un point du segment de rang 1 dans un repère de référence est la position du point dans le repère de référence.

**18.** Procédé de reproduction de mouvement d'une structure constituée de N segments solides successifs articulés les uns par rapport aux autres à partir d'un segment de rang 1 jusqu'à un segment de rang N, N étant un nombre entier supérieur ou égal à 2, le segment de rang n (n = 2, ..., N) étant articulé avec le segment de rang n-1 au niveau d'un point d'articulation $p_n$, **caractérisé en ce qu'**il met en oeuvre :

- un procédé de capture de mouvement selon l'une quelconque des revendications 14 à 17, et
- un calcul, à chaque instant $t_k$ :

a) du vecteur d'accélération absolue $\vec{a}_1$, dans le repère de référence, à partir de la mesure apte à restituer un vecteur d'accélération absolue $\vec{a}_1$,

b) du vecteur d'orientation $\vec{\Theta}_1$ du segment de rang 1 dans le repère de référence, à partir du vecteur d'accélération absolue $\vec{a}_1$ et de la mesure représentative ($M_1$) du vecteur orientation ($\vec{\Theta}_1$) du segment de rang 1 ;

c) d'un vecteur d'accélération $\vec{a}_n$ (n ≥ 2) du point d'articulation $p_n$ dans le repère de référence, à partir de l'équation :

$$\vec{a}_n = \vec{a}_{n-1} + \left(\frac{d\vec{\omega}_{n-1}}{dt}\right)\Lambda \overrightarrow{L_{n-1}} + \vec{\omega}_{n-1}\Lambda\left(\vec{\omega}_{n-1}\Lambda\overrightarrow{L_{n-1}}\right)$$

où $\vec{\omega}_n$ = d($\vec{\Theta}_n$)/dt, $\vec{L}_n$ étant un vecteur orienté du point d'articulation $p_{n-1}$ vers le point d'articulation $p_n$ et dont le module a pour valeur la distance qui sépare le point d'articulation $p_n$ du point d'articulation $p_{n-1}$, la mesure supplémentaire représentative du vecteur orientation $\vec{\Theta}_n$ étant délivrée par des moyens de mesure fixés, sur le segment de rang n, sensiblement au niveau du point d'articulation $p_n$; et

d) d'un vecteur d'orientation $\vec{\Theta}_n$ (n ≥ 2) du segment de rang n à partir du vecteur d'accélération $\vec{a}_n$ et de la mesure représentative ($M_n$) de l'orientation du segment de rang n.

**19.** Procédé de reproduction de mouvement d'une structure constituée de N segments solides successifs articulés les uns par rapport aux autres à partir d'un segment de rang 1 jusqu'à un segment de rang N, N étant un nombre entier supérieur ou égal à 2, le segment de rang n (n = 2, ..., N) étant articulé avec le segment de rang n-1 au niveau d'un point d'articulation $p_n$, **caractérisé en ce qu'**il comprend :

- un procédé de capture de mouvement selon l'une quelconque des revendications 14 à 17 ; et
- un calcul, à chaque instant $t_k$ :

a) du vecteur d'accélération absolue $\vec{a}_1$ dans le repère de référence, à partir de l'information délivrée par les premiers moyens,

b) du vecteur d'orientation $\vec{\Theta}_1$ du segment de rang 1 dans le repère de référence, à partir du vecteur d'accélération absolue $\vec{a}_1$ et de la mesure représentative ($M_1$) du vecteur orientation ($\vec{\Theta}_1$) du segment de rang 1 ;

c) un vecteur d'accélération $\vec{a}_n$ (n ≥ 2) du point d'articulation $p_n$ dans le repère de référence, à partir de l'équation :

$$\vec{a}_n = \vec{a}_{n-1} + \left(\frac{d\vec{\omega}_{n-1}}{dt}\right)\Lambda \overrightarrow{L_{n-1}} + \vec{\omega}_{n-1}\Lambda\left(\vec{\omega}_{n-1}\Lambda\overrightarrow{L_{n-1}}\right)$$

où $\vec{\omega}_n$ = d($\vec{\Theta}_n$)/dt, $\vec{L}_n$ étant un vecteur orienté du point d'articulation $p_{n-1}$ vers le point d'articulation $p_n$ et dont le module a pour valeur la distance qui sépare le point d'articulation $p_n$ du point d'articulation $p_{n-1}$ ; et

d) le vecteur d'orientation $\vec{\Theta}_n$ (n ≥ 2) et un vecteur d'accélération $\vec{b}_n$ du point de mesure des moyens de mesure supplémentaire fixés sur le segment de rang n à partir du vecteur d'accélération $\vec{a}_n$, de la mesure représentative ($M_n$) de l'orientation du segment de rang n, et des vecteurs d'orientation du segment de rang n à au moins deux instants qui précèdent l'instant $t_k$, avec $\vec{b}_n$ tel que :

$$\vec{b}_n = \vec{a}_n + \left(\frac{d\vec{\omega}_n}{dt}\right) \wedge \vec{D}_n + \vec{\omega}_n \wedge \left(\vec{\omega}_n \wedge \vec{D}_n\right)$$

où $\vec{D}_n$ est un vecteur orienté du point d'articulation $p_n$ vers les moyens de mesure supplémentaires du segment de rang n et dont le module est sensiblement égal à la distance qui sépare le point d'articulation $p_n$ des moyens de mesure supplémentaires du segment de rang n.

**Claims**

1. Motion capture device of a structure consisting of N successive solid segments articulated with respect to one another from a segment of rank 1 as far as a segment of rank N, N being an integer number greater than or equal to 2, the segment of rank n (n = 2, ..., N) being articulated with the segment of rank n-1 at an articulation point $p_n$, **characterised in that** it comprises:

   - first means (ML) that deliver information able to restore an absolute acceleration vector $\vec{a}_1$ of a point on the segment of rank 1 in a reference frame forming a reference, at successive times $t_k$, k being an integer number greater than or equal to 1,
   - second measuring means (MD$_1$) fixed to the segment of rank 1 and which deliver, at each time $t_k$, a measurement (M$_1$) representing an orientation vector $\vec{\Theta}_1$ of the segment of rank 1 in the reference frame, and
   - supplementary measurement means (MD$_n$) fixed to each segment of rank n (n = 2, ..., N), and which deliver, at each time $t_k$, a measurement representing an orientation vector $\vec{\Theta}_n$ of the segment of rank n.

2. Motion capture device according to claim 1, in which the second measuring means (MD$_1$) and the supplementary measuring means (MD$_n$) consist of an accelerometer and a sensor that delivers a measurement of a uniform physical field present in the space where the structure moves and with a known direction in the reference frame.

3. Motion capture device according to claim 2, in which the second measuring means (MD$_1$) and the supplementary measuring means (MD$_n$) further comprise at least one gyrometric axis.

4. Motion capture device according to either one of claims 2 or 3, in which the sensor that delivers a measurement of a uniform physical field of known direction in the reference frame is a magnetometer.

5. Motion capture device according to either one of claims 2 or 3, in which the sensor that delivers a measurement of a uniform physical field of known direction in the reference frame is a photoelectric cell.

6. Device according to any one of the preceding claims, in which the first means (ML) are measuring means consisting of a velocity measurer so that the data item able to restore an absolute acceleration vector of the segment of rank 1 is the velocity of the point.

7. Device according to any one of claims 1 to 5, in which the first means (ML) are measuring means consisting of a position measurer so that the data item able to restore an absolute acceleration vector of a point on the segment of rank 1 is the position of the point.

8. Device for reproducing the motion of a structure consisting of N successive solid segments articulated with respect to one another from a segment of rank 1 as far as a segment of rank N, N being an integer number greater or equal to 2, the segment of rank n (n = 2, ..., N) being articulated with the segment of rang n-1 at an articulation point $p_n$, **characterised in that** it comprises:

   - a motion capture device according to any one of claims 1 to 7 in which the supplementary measurement means (MD$_n$) of a segment of rank n are positioned close to the articulation point $p_n$ so that the distance that separates the supplementary measuring means (MD$_n$) of a segment of rank n from the articulation point $p_n$ is considered to be zero, and
   - calculation means (3) that calculate, at each time $t_k$:

      a) the absolute acceleration vector $\vec{a}_1$ in the reference frame, from the information delivered by the first means,

b) the orientation vector $\overrightarrow{\Theta}_1$ of the segment of rank 1 in the reference frame, from the absolute acceleration vector $\overrightarrow{a}_1$ and the measurement ($M_1$) representing the orientation vector ($\overrightarrow{\Theta}_1$) of the segment of rank 1;

c) an acceleration vector $\overrightarrow{a}_n$ ($n \geq 2$) of the articulation point $p_n$ in the reference frame, from the equation:

$$\vec{a}_n = \vec{a}_{n-1} + \left(\frac{d\vec{\omega}_{n-1}}{dt}\right)\Lambda \overrightarrow{L_{n-1}} + \vec{\omega}_{n-1}\Lambda\left(\vec{\omega}_{n-1}\Lambda\overrightarrow{L_{n-1}}\right)$$

where $\overrightarrow{\omega}_n = d(\overrightarrow{\Theta}_n)/dt$, $\overrightarrow{L}_n$ being a vector oriented from the articulation point $p_{n-1}$ to the articulation point $p_n$ and whose modulus has as its value the distance that separates the articulation point $p_n$ from the articulation point $p_{n-1}$; and

d) the orientation vector $\overrightarrow{\Theta}_n$ ($n \geq 2$) of the segment of rank n from the acceleration vector $\overrightarrow{a}_n$ and the measurement ($M_n$) representing the orientation of the segment of rank n.

9. Device for reproducing the motion of a structure consisting of N successive solid segments articulated with respect to one another from a segment of rank 1 as far as a segment of rank N, N being an integer number greater than or equal to 2, the segment of rank n (n = 2, ..., N) being articulated with the segment of rank n-1 at an articulation point $p_n$, **characterised in that** it comprises:

- a motion capture device according to any one of claims 1 to 7 in which the supplementary measuring means ($MD_n$) of a segment of rank n are distant from the articulation point $p_n$, and
- calculation means (3) that calculate, at each time $t_k$:

a) the absolute acceleration vector $\overrightarrow{a}_1$ in the reference frame, from the information delivered by the first means,

b) the orientation vector $\overrightarrow{\Theta}_1$ of the segment of rank 1 in the reference frame, from the absolute acceleration vector $\overrightarrow{a}_1$ and the measurement ($M_1$) representing the orientation vector $\overrightarrow{\Theta}_1$ of the segment of rank 1;

c) an acceleration vector $\overrightarrow{a}_n$ ($n \geq 2$) of the articulation point $p_n$ in the reference frame, from the equation:

$$\vec{a}_n = \vec{a}_{n-1} + \left(\frac{d\vec{\omega}_{n-1}}{dt}\right)\Lambda \overrightarrow{L_{n-1}} + \vec{\omega}_{n-1}\Lambda\left(\vec{\omega}_{n-1}\Lambda\overrightarrow{L_{n-1}}\right)$$

where $\overrightarrow{\omega}_n = d(\overrightarrow{\Theta}_n)/dt$, $\overrightarrow{L}_n$ being a vector oriented from the articulation point $p_{n-1}$ to the articulation point $p_n$ and whose modulus has as its value the distance that separates the articulation point $p_n$ from the articulation point $p_{n-1}$; and

d) the orientation vector $\overrightarrow{\Theta}_n$ ($n \geq 2$) and an acceleration vector $\overrightarrow{b}_n$ of the measuring point of the supplementary measuring means fixed on the segment of rank n from the acceleration vector $\overrightarrow{a}_n$, the measurement ($M_n$) representing the orientation of the segment of rank n, and the orientation vectors of the segment of rank n at at least two times that precede the time $t_k$, with $\overrightarrow{b}_n$ such that:

$$\vec{b}_n = \vec{a}_n + \left(\frac{d\vec{\omega}_n}{dt}\right) \wedge \vec{D}_n + \vec{\omega}_n \wedge \left(\vec{\omega}_n \wedge \vec{D}_n\right)$$

where $\overrightarrow{D}_n$ is a vector oriented from the articulation point $p_n$ to the supplementary measuring means of the segment of rank n and whose modulus is substantially equal to the distance that separates the articulation point $p_n$ from the supplementary measuring means of the segment of rank n.

10. Motion reproduction device according to either one of claims 8 or 9, in which radio transmission means transmit elementary electrical signals ($RD_n$, $RL_m$) representing the measurements delivered by the first measuring means (ML) and the second measuring means ($MD_n$) to the calculation means (3).

**11.** Motion reproduction device according to claim 10, in which the transmission means comprise an intermediate unit (DEM) that receives the elementary electrical signals ($RD_1$, ..., $RD_X$, $RL_1$, ..., $RL_Y$) and that retransmits an electrical signal (RF) representing elementary electrical signals to the calculation means (3).

**12.** Device according to any one of the preceding claims, in which storage means store the measurements delivered by the first measuring means (ML) and the second measuring means ($MD_n$).

**13.** Device according to claim 12, in which the storage means are placed on the structure.

**14.** Method of capturing the motion of a structure consisting of N successive solid segments articulated with respect to one another from a segment of rank 1 as far as a segment of rank N, N being an integer number greater than or equal to 2, the segment of rang n (n = 2, ..., N) being articulated with the segment of rank n-1 at an articulation point $p_n$, **characterised in that** it comprises:

- at least one determination of an item of information able to restore an absolute acceleration vector $\vec{a}_1$ of a point on the segment of rank 1 in a reference frame, at successive times $t_k$, k being an integer number greater than or equal to 1,
- at least one measurement representing an orientation vector $\vec{\Theta}_1$ of the segment of rank 1 in the reference frame, at each of the successive times $t_k$, and
- for each segment of rank n, at least one supplementary measurement of an orientation vector $\vec{\Theta}_n$ of the segment of rank n in the reference frame, at each of the successive times $t_k$.

**15.** Motion capture method according to claim 14, in which the measurement representing the orientation vector $\vec{\Theta}_1$ of the segment of rank 1 in the reference frame and the measurement representing the orientation vector $\vec{\Theta}_n$ of the segment of rank n are each a measurement of a uniform field present in the space where the structure moves and with a known direction in the reference frame.

**16.** Motion capture method according to claim 14 or 15, in which the information able to restore an absolute acceleration vector $\vec{a}_1$ of a point on the segment of rank 1 in a reference frame is the velocity of the point in the reference frame.

**17.** Motion capture method according to claim 14 or 15, in which the information able to restore an absolute acceleration vector $\vec{a}_1$ of a point on the segment of rank 1 in a reference frame is the position of the point in the reference frame.

**18.** Method for reproducing the motion of a structure consisting of N successive solid segments articulated with respect to one another from a segment of rank 1 as far as a segment of rank N, N being an integer number greater than or equal to 2, the segment of rang n (n = 2, ..., N) being articulated with the segment of rank n-1 at an articulation point $p_n$, **characterised in that** it uses:

- a motion capture method according to any one of claims 14 to 17, and
- a calculation, at each time $t_k$:

a) of the absolute acceleration vector $\vec{a}_1$ in the reference frame, from the measurement able to restore an absolute acceleration vector $\vec{a}_1$,
b) of the orientation vector $\vec{\Theta}_1$ of the segment of rank 1 in the reference frame, from the absolute acceleration vector $\vec{a}_1$ and the measurement ($M_1$) representing the orientation vector ($\vec{\Theta}_1$) of the segment of rank 1;
c) of an acceleration vector $\vec{a}_n$ (n ≥ 2) of the articulation point $p_n$ in the reference frame, from the equation:

$$\vec{a}_n = \vec{a}_{n-1} + \left(\frac{d\vec{\omega}_{n-1}}{dt}\right)\Lambda\ \overrightarrow{L_{n-1}} + \vec{\omega}_{n-1}\Lambda\left(\vec{\omega}_{n-1}\Lambda\overrightarrow{L_{n-1}}\right)$$

where $\vec{\omega}_n = d(\vec{\Theta}_n)/dt$, $\overrightarrow{L_n}$ being a vector oriented from the articulation point $p_{n-1}$ towards the articulation point $p_n$ and whose modulus has as its value the distance that separates the articulation point $p_n$ from the articulation point $p_{n-1}$, the supplementary measurement representing the orientation vector $\vec{\Theta}_n$ being delivered by fixed measuring means, on the segment of rank n, substantially at the articulation point $p_n$; and
d) of an orientation vector $\vec{\Theta}_n$ (n ≥ 2) of the segment of rank n from the acceleration vector $\vec{a}_n$ and the

measurement ($M_n$) representing the orientation of the segment of rank n.

**19.** Method for reproducing motion of a structure consisting of N successive solid segments articulated with respect to one another from a segment of rank 1 as far as a segment of rank N, N being an integer number greater or equal to 2, segment of rank n (n = 2, ..., N) being articulated with the segment of rang n-1 at an articulation point $p_n$, **characterised in that** it comprises:

- a motion capture method according to any one of claims 14 to 17; and
- a calculation, at each time $t_k$:

a) of the absolute acceleration vector $\vec{a}_1$ in the reference frame, from the information delivered by the first means,

b) of the orientation vector $\vec{\Theta}_1$ of the segment of rank 1 in the reference frame, from the absolute acceleration vector $\vec{a}_1$ and the measurement ($M_1$) representing the orientation vector $\vec{\Theta}_1$ of the segment of rank 1;

c) of an acceleration vector $\vec{a}_n$(n > 2) of the articulation $p_n$ in the reference frame, from the equation:

$$\vec{a}_n = \vec{a}_{n-1} + \left(\frac{d\vec{\omega}_{n-1}}{dt}\right)\Lambda\,\overrightarrow{L_{n-1}} + \vec{\omega}_{n-1}\Lambda\left(\vec{\omega}_{n-1}\Lambda\overrightarrow{L_{n-1}}\right)$$

where $\vec{\omega}_n = d(\vec{\Theta}_n)/dt$, $\vec{L}_n$ being a vector oriented from the articulation point $p_{n-1}$ to the articulation point $p_n$ and whose modulus has as its value the distance that separates the articulation point $p_n$ from the articulation point $p_{n-1}$; and

d) the orientation vector $\vec{\Theta}_n$ (n ≥ 2) and an acceleration vector $\vec{b}_n$ of the measuring point of the supplementary measuring means fixed on the segment of rank n from the acceleration vector $\vec{a}_n$, the measurement ($M_n$) representing the orientation of the segment of rank n, and orientation vectors of the segment of rank n at at least two times that precede the time $t_k$, with $\vec{b}_n$ such that:

$$\vec{b}_n = \vec{a}_n + \left(\frac{d\vec{\omega}_n}{dt}\right)\wedge\vec{D}_n + \vec{\omega}_n\wedge\left(\vec{\omega}_n\wedge\vec{D}_n\right)$$

where $\vec{D}_n$ is a vector oriented from the articulation point $p_n$ to the supplementary measuring means of the segment of rank n and whose modulus is substantially equal to the distance that separates the articulation point $p_n$ from the supplementary measuring means of the segment of rank n.

**Patentansprüche**

**1.** Vorrichtung zur Erfassung der Bewegung einer Struktur, gebildet durch N sukzessive feste Elemente, gelenkig miteinander verbunden von einem Segment des Rangs 1 bis zu einem Segment des Rangs N, wobei N eine ganze Zahl größer oder gleich 2 ist und das Segment des Rangs n (n = 2, ..., N) in Höhe eines Gelenkpunkts $p_n$ mit dem Segment n-1 gelenkig verbunden ist, **dadurch gekennzeichnet, dass** sie umfasst:

- erste Einrichtungen (ML), die eine Information liefern, fähig einen Absolutbeschleunigungs-Vektor $\vec{a}_1$ eines Punkts des Segments des Rangs 1 in einem Bezugssystem zu sukzessiven Zeitpunkten $t_k$ wiederzugeben, wobei k eine ganze Zahl größer oder gleich 1 ist,
- zweite Messeinrichtungen ($MD_1$), befestigt auf dem Segment des Rangs 1, die zu jedem Zeitpunkt $t_k$ einen Messwert ($M_1$) liefern, der repräsentativ ist für einen Orientierungsvektor $\vec{\Theta}_1$ des Segments des Rangs 1 in dem Bezugssystem, und
- zusätzliche Messeinrichtungen ($MD_n$), befestigt auf jedem Segment des Rangs n (n = 2, ..., N), die zu jedem Zeitpunkt $t_k$ einen für einen Orientierungsvektor $\vec{\Theta}_n$ des Segments des Rangs n repräsentativen Messwert liefern.

2. Bewegungserfassungsvorrichtung nach Anspruch 1, bei der die zweiten Messeinrichtungen ($MD_1$) und die zusätzlichen Messeinrichtungen ($MD_n$) gebildet werden durch einen Beschleunigungsmesser und einen Sensor, der einen Messwert eines gleichmäßigen physikalischen Feldes liefert, das in dem Raum, in dem sich die Struktur in dem Bezugssystem bewegt, präsent ist und dessen Richtung bekannt ist.

3. Bewegungserfassungsvorrichtung nach Anspruch 2, bei der die zweiten Messeinrichtungen ($MD_1$) und die zusätzlichen Messeinrichtungen ($MD_n$) außerdem eine gyrometrische Achse umfassen.

4. Bewegungserfassungsvorrichtung nach einem der Ansprüche 2 oder 3, bei der der Sensor, der einen Messwert eines gleichmäßigen physikalischen Feldes von bekannter Richtung in dem Bezugssystem liefert, ein Magnetometer ist.

5. Bewegungserfassungsvorrichtung nach einem der Ansprüche 2 oder 3, bei der der Sensor, der einen Messwert eines gleichmäßigen physikalischen Feldes von bekannter Richtung in dem Bezugssystem liefert, eine fotoelektrische Zelle ist.

6. Vorrichtung nach einem der vorangehenden Ansprüche, bei der die ersten Einrichtungen (ML) Messeinrichtungen sind, gebildet durch einen Geschwindigkeitsmesser, so dass die einen Absolutbeschleunigungs-Vektor des Segments des Rangs 1 wiedergebende Größe die Geschwindigkeit des Punkts ist.

7. Vorrichtung nach einem der Ansprüche 1 bis 5, bei der die ersten Einrichtungen (ML) Messeinrichtungen sind, gebildet durch einen Positionsmesser, so dass die einen Absolutbeschleunigungs-Vektor des Segments des Rangs 1 wiedergebende Größe die Position des Punkts ist.

8. Vorrichtung zur Reproduktion der Bewegung einer Struktur, gebildet durch N sukzessive feste Elemente, gelenkig miteinander verbunden von einem Segment des Rangs 1 bis zu einem Segment des Rangs N, wobei N eine ganze Zahl größer oder gleich 2 ist und das Segment des Rangs n (n = 2, ..., N) in Höhe eines Gelenkpunkts $p_n$ mit dem Segment n-1 gelenkig verbunden ist,
**dadurch gekennzeichnet, dass** sie umfasst:

   - eine Bewegungserfassungsvorrichtung nach einem der Ansprüche 1 bis 7, bei der die zusätzlichen Messeinrichtungen ($MD_n$) eines Segments des Rangs n in der Nähe des Gelenkpunkts $p_n$ positioniert sind, so dass der Abstand, der die zusätzlichen Messeinrichtungen ($MD_n$) eines Segments des Rangs n von dem Gelenkpunkt $p_n$ trennt, als null betrachtet wird, und
   - Recheneinrichtungen (3), die zu jedem Zeitpunkt $t_k$ berechnen:

   a) den Absolutbeschleunigungs-Vektor $\vec{a}_1$ in dem Bezugssystem aufgrund der durch die ersten Einrichtungen gelieferten Information,
   b) den Orientierungsvektor $\vec{\Theta}_1$ des Segments des Rangs 1 in dem Bezugssystem aufgrund des Absolutbeschleunigungs-Vektors $\vec{a}_1$ und des für den Orientierungsvektor ($\vec{\Theta}_1$) des Segments des Rangs 1 repräsentativen Messwerts ($M_1$);
   c) einen Beschleunigungsvektor $\vec{a}_n$ (n ≥ 2) des Gelenkpunkts $p_n$ in dem Bezugssystem aufgrund der Gleichung:

$$\vec{a}_n = \vec{a}_{n-1} + \left(\frac{d\vec{\omega}_{n-1}}{dt}\right)\varLambda\vec{L}_{n-1} + \vec{\omega}_{n-1}\varLambda\left(\vec{\omega}_{n-1}\varLambda\vec{L}_{n-1}\right)$$

   wo $\vec{\omega}_{n-1} = d(\vec{\Theta}_n)/dt$ und $\vec{L}_n$ ein vom Gelenkpunkt $p_{n-1}$ in Richtung Gelenkpunkt $p_n$ orientierter Vektor ist, dessen Modul als Wert den Abstand hat, der den Gelenkpunkt $p_n$ vom Gelenkpunkt $p_{n-1}$ trennt; und
   d) den Orientierungsvektor $\vec{\Theta}_n$ (n ≥ 2) des Segments des Rangs n aufgrund des Beschleunigungsvektors $\vec{a}_n$ und des für die Orientierung des Segments des Rangs n repräsentativen Messwerts ($M_n$).

9. Vorrichtung zur Reproduktion der Bewegung einer Struktur, gebildet durch N sukzessive feste Elemente, gelenkig miteinander verbunden von einem Segment des Rangs 1 bis zu einem Segment des Rangs N, wobei N eine ganze Zahl größer oder gleich 2 ist und das Segment des Rangs n (n = 2, ..., N) in Höhe eines Gelenkpunkts $p_n$ mit dem Segment n-1 gelenkig verbunden ist,

**dadurch gekennzeichnet, dass** sie umfasst:

- eine Bewegungserfassungsvorrichtung nach einem der Ansprüche 1 bis 7, bei der die zusätzlichen Messeinrichtungen ($MD_n$) eines Segments des Rangs n von dem Gelenkpunkt $p_n$ entfernt bzw. weiter entfernt sind, und
- Recheneinrichtungen (3), die zu jedem Zeitpunkt $t_k$ berechnen:

a) den Absolutbeschleunigungs-Vektor $\vec{a}_1$ in dem Bezugssystem aufgrund der durch die ersten Einrichtungen gelieferten Information,
b) den Orientierungsvektor $\vec{\Theta}_1$ des Segments des Rangs 1 in dem Bezugssystem aufgrund des Absolutbeschleunigungs-Vektors $\vec{\alpha}_1$ und des für den Orientierungsvektor ($\vec{\Theta}_1$) des Segments des Rangs 1 repräsentativen Messwerts ($M_1$);
c) einen Beschleunigungsvektor $\vec{a}_n$ ($n \geq 2$) des Gelenkpunkts $p_n$ in dem Bezugssystem aufgrund der Gleichung:

$$\vec{a}_n = \vec{a}_{n-1} + \left(\frac{d\vec{\omega}_{n-1}}{dt}\right)\Lambda\vec{L}_{n-1} + \vec{\omega}_{n-1}\Lambda\left(\vec{\omega}_{n-1}\Lambda\vec{L}_{n-1}\right)$$

wo $\vec{\omega}_n = d(\vec{\Theta}_n)/dt$ und $\vec{L}_n$ ein vom Gelenkpunkt $p_{n-1}$ in Richtung Gelenkpunkt $p_n$ orientierter Vektor ist, dessen Modul als Wert den Abstand hat, der den Gelenkpunkt $p_n$ vom Gelenkpunkt $p_{n-1}$ trennt; und
d) den Orientierungsvektor $\vec{\Theta}_n$ ($n \geq 2$) und einen Beschleunigungsvektor $\vec{b}_n$ des Messpunkts der auf dem Segment des Rangs n befestigten zusätzlichen Messeinrichtungen aufgrund des Beschleunigungsvektors $\vec{a}_n$ der Messung bzw. des Messwerts ($M_n$), repräsentativ für die Orientierung des Segments des Rangs n, und Orientierungsvektoren des Segment des Rangs n zu wenigstens zwei dem Zeitpunkt $t_k$ vorangehenden Zeitpunkten, mit $\vec{b}_n$ so dass:

$$\vec{b}_n = \vec{a}_n + \left(\frac{d\vec{\omega}_n}{dt}\right)\wedge\vec{D}_n + \vec{\omega}_n \wedge\left(\vec{\omega}_n \wedge\vec{D}_n\right)$$

wo $\vec{D}_n$ ein von dem Gelenkpunkt $p_n$ in Richtung der zusätzlichen Messeinrichtungen des Segments des Rangs n orientierter Vektor ist, dessen Modul im Wesentlichen gleich dem Abstand ist, der den Gelenkpunkt $p_n$ von den zusätzlichen Messeinrichtungen des Segments des Rangs n trennt.

10. Vorrichtung zur Reproduktion der Bewegung einer Struktur nach einem der Ansprüche 8 oder 9, bei der radioelektrische Übertragungseinrichtungen elementare elektrische Signale ($RD_n$, $RL_m$) übertragen, repräsentativ für die durch die ersten Messeinrichtungen (ML) und die zweiten Messeinrichtungen ($MD_n$) den Recheneinrichtungen (3) gelieferten Messwerte.

11. Vorrichtung zur Reproduktion der Bewegung einer Struktur nach Anspruch 10, bei der die Übertragungseinrichtungen eine intermediäre Einheit (DEM) umfassen, die die elementaren elektrischen Signale ($RD_1$, ..., $RD_x$, $RL_1$, ..., $RL_y$) empfangen und in Richtung Recheneinrichtungen (3) ein für die elementaren elektrischen Signale repräsentatives elektrisches Signal (RF) wiederaussenden.

12. Vorrichtung nach einem der vorangehenden Ansprüche, bei der die Speichereinrichtungen die durch die ersten Messeinrichtungen (ML) und die zweiten Messeinrichtungen ($MD_n$) gelieferten Messwerte einspeichern.

13. Vorrichtung nach Anspruch 12, bei der die Speichereinrichtungen sich auf der Struktur befinden.

14. Verfahren zur Erfassung der Bewegung einer Struktur, gebildet durch N sukzessive feste Elemente, gelenkig miteinander verbunden von einem Segment des Rangs 1 bis zu einem Segment des Rangs N, wobei N eine ganze Zahl größer oder gleich 2 ist und das Segment des Rangs n (n = 2, ..., N) in Höhe eines Gelenkpunkts $p_n$ mit dem Segment n-1 gelenkig verbunden ist, **dadurch gekennzeichnet, dass** es umfasst:

- wenigstens eine Determination einer Information, fähig einen Absolutbeschleunigungsvektor $\vec{a}_1$ eines Punkts

des Segments des Rangs 1 in einem Bezugssystem wiederzugeben, zu sukzessiven Zeitpunkten $t_k$, wobei k eine ganze Zahl größer oder gleich 1 ist,
- zu jedem der sukzessiven Zeitpunkte $t_k$ wenigstens einen Messwert, der repräsentativ ist für einen Orientierungsvektor $\vec{\Theta}_1$ des Segments des Rangs 1 in dem Bezugssystem, und
- zu jedem der sukzessiven Zeitpunkte $t_k$ wenigstens einen zusätzlichen Messwert eines Orientierungsvektors $\vec{\Theta}_n$ des Segments des Rangs n in dem Bezugssystem.

**15.** Bewegungserfassungsverfahren nach Anspruch 14, bei der sowohl der Messwert, der repräsentativ ist für den Orientierungsvektor $\vec{\Theta}_1$ des Segments des Rangs 1 in dem Bezugssystem, als auch der Messwert, der repräsentativ ist für den Orientierungsvektor $\vec{\Theta}_n$ des Segments des Rangs n in dem Bezugssystem, Messwerte eines gleichmäßigen Feldes in dem Raum sind, in dem sich die Struktur mit bekannter Richtung in dem Bezugssystem bewegt.

**16.** Bewegungserfassungsverfahren nach Anspruch 14 oder 15, bei dem die zur Wiedergabe eines Absolutbeschleunigungsvektor $\vec{a}_1$ eines Punkts des Segments des Rangs 1 in einem Bezugssystem fähige Information die Geschwindigkeit des Punkts in dem Bezugssystem ist.

**17.** Bewegungserfassungsverfahren nach Anspruch 14 oder 15, bei dem die zur Wiedergabe eines Absolutbeschleunigungsvektor $\vec{a}_1$ eines Punkts des Segments des Rangs 1 in einem Bezugssystem fähige Information die Position des Punkts in dem Bezugssystem ist.

**18.** Verfahren zur Reproduktion der Bewegung einer Struktur, gebildet durch N sukzessive feste Elemente, gelenkig miteinander verbunden von einem Segment des Rangs 1 bis zu einem Segment des Rangs N, wobei N eine ganze Zahl größer oder gleich 2 ist und das Segment des Rangs n (n = 2, ..., N) in Höhe eines Gelenkpunkts $p_n$ mit dem Segment n-1 gelenkig verbunden ist, **dadurch gekennzeichnet, dass** es anwendet:

- ein Bewegungserfassungsverfahren nach einem der Ansprüche 14 bis 17, und
- eine Berechnung zu jedem Zeitpunkt $t_k$:

a) des Absolutbeschleunigungsvektors $\vec{a}_1$ in dem Bezugssystem aufgrund der zur Wiedergabe eines Absolutbeschleunigungsvektors $\vec{a}_1$ fähigen Messung,
b) des Orientierungsvektors $\vec{\Theta}_1$ des Segments des Rangs 1 in dem Bezugssystem aufgrund des Absolutbeschleunigungsvektors $\vec{a}_1$ und der Messung bzw. des Messwerts ($M_1$) des Orientierungsvektors $\vec{\Theta}_1$ des Segments des Rangs 1;
c) eines Beschleunigungsvektors $\vec{a}_n$ (n ≥ 2) des Gelenkpunkts $p_n$ in dem Bezugssystem aufgrund der Gleichung:

$$\vec{a}_n \;=\; \vec{a}_{n-1} \;+\; \left(\frac{d\vec{\omega}_{n-1}}{dt}\right)A\vec{L}_{n-1} \;+\; \vec{\omega}_{n-1}A\!\left(\vec{\omega}_{n-1}A\vec{L}_{n-1}\right)$$

wo $\vec{\omega}_n = d(\vec{\Theta}_n)/dt$ und $\vec{L}_n$ ein vom Gelenkpunkt $p_{n-1}$ in Richtung Gelenkpunkt $p_n$ orientierter Vektor ist, dessen Modul als Wert den Abstand hat, der den Gelenkpunkt $p_n$ vom Gelenkpunkt $p_{n-1}$ trennt, wobei der für den Orientierungsvektor $\vec{\Theta}_n$ repräsentative zusätzliche Messwert geliefert wird durch Messeinrichtungen, die auf dem Segment des Rangs n befestigt sind, im Wesentlichen in Höhe der Gelenkpunkts $p_n$; und
d) eines Orientierungsvektors $\vec{\Theta}_n$ (n ≥ 2) des Segments des Rangs n aufgrund des Beschleunigungsvektors $\vec{a}a_n$ und des für die Orientierung des Segments des Rangs n repräsentativen Messwerts ($M_n$).

**19.** Verfahren zur Reproduktion der Bewegung einer Struktur, gebildet durch N sukzessive feste Elemente, gelenkig miteinander verbunden von einem Segment des Rangs 1 bis zu einem Segment des Rangs N, wobei N eine ganze Zahl größer oder gleich 2 ist und das Segment des Rangs n (n = 2, ..., N) in Höhe eines Gelenkpunkts $p_n$ mit dem Segment n-1 gelenkig verbunden ist, **dadurch gekennzeichnet, dass** es umfasst:

- ein Bewegungserfassungsverfahren nach einem der Ansprüche 14 bis 17, und
- eine Berechnung zu jedem Zeitpunkt $t_k$:

a) des Absolutbeschleunigungsvektors $\vec{a}_1$ in dem Bezugssystem aufgrund der durch die ersten Einrichtungen gelieferten Information,

b) des Orientierungsvektors $\vec{\Theta}_1$ des Segments des Rangs 1 in dem Bezugssystem aufgrund des Absolutbeschleunigungsvektors $\vec{a}_1$ und der Messung bzw. des Messwerts ($M_1$) des Orientierungsvektors $\vec{\Theta}_1$ des Segments des Rangs 1;

c) eines Beschleunigungsvektors $\vec{a}_n$ ($n \geq 2$) des Gelenkpunkts $p_n$ in dem Bezugssystem aufgrund der Gleichung:

$$\vec{a}_n \;=\; \vec{a}_{n-1} \;+\; \left(\frac{d\vec{\omega}_{n-1}}{dt}\right)\Lambda \vec{L}_{n-1} \;+\; \vec{\omega}_{n-1}\Lambda\left(\vec{\omega}_{n-1}\Lambda\vec{L}_{n-1}\right)$$

wo $\vec{\omega}_n = d(\vec{\Theta}_n)/dt$ und $\vec{L}_n$ ein vom Gelenkpunkt $p_{n-1}$ in Richtung Gelenkpunkt $p_n$ orientierter Vektor ist, dessen Modul als Wert den Abstand hat, der den Gelenkpunkt $p_n$ vom Gelenkpunkt $p_{n-1}$ trennt; und

d) des Orientierungsvektors $\vec{\Theta}_n$ ($n \geq 2$) und eines Beschleunigungsvektors $\vec{b}_n$ des Messpunkts der auf dem Segment des Rangs n befestigten zusätzlichen Messeinrichtungen aufgrund des Beschleunigungsvektors $\vec{a}_n$ des für die Orientierung des Segments des Rangs n repräsentativen Messwerts ($M_n$), und Orientierungsvektoren des Segment des Rangs n zu wenigstens zwei dem Zeitpunkt $t_k$ vorangehenden Zeitpunkten, mit $\vec{b}_n$ so dass:

$$\vec{b}_n = \vec{a}_n + \left(\frac{d\vec{\omega}_n}{dt}\right)\wedge\vec{D}_n + \vec{\omega}_n \wedge \left(\vec{\omega}_n \wedge \vec{D}_n\right)$$

wo $\vec{D}_n$ ein von dem Gelenkpunkt $p_n$ in Richtung der zusätzlichen Messeinrichtungen des Segments des Rangs n orientierter Vektor ist, dessen Modul im Wesentlichen gleich dem Abstand ist, der den Gelenkpunkt $p_n$ von den zusätzlichen Messeinrichtungen des Segments des Rangs n trennt.

FIG. 1

FIG. 2

FIG. 3

$\vec{a}_{n-1}$    $\vec{\theta}_{n-1}$    $\vec{L}_{n-1}$

EQUATION (4)    ⟋ 1

Mn    $\vec{a}_n$    G    H

EQUATION (5)    ⟋ 2

$\vec{a}_n$    $\vec{\theta}_n$

# FIG. 4A

$\vec{a}_{n-1}$    $\vec{\theta}_{n-1}$    $\vec{L}_{n-1}$

$\vec{\theta}_n(t_k - 2)$

EQUATION (4)

$\vec{\theta}_n(t_k - 1)$

$Mn(t_k)$    $\vec{a}_n(t_k)$    G    H

EQUATION (6)

$\vec{a}_n(t_k)$    $\vec{\theta}_n(t_k)$

# FIG. 4B

FIG. 5A

FIG. 5B

| $t_k$ | $n=1$ | $n=2$ | $n=3$ | $n=4$ | $n=5$ |
|---|---|---|---|---|---|
| $t_{11}$ | $a_1,\theta_1,\dot\theta_1,\ddot\theta_1$ | $a_2,\theta_2,\dot\theta_2,\ddot\theta_2$ | $a_3,\theta_3,\dot\theta_3,\ddot\theta_3$ | $a_4,\theta_4,\dot\theta_4,\ddot\theta_4$ | $a_5,\dot\theta_5,\dot\theta_5,\ddot\theta_5$ |
| $t_{10}$ | $a_1,\theta_1,\dot\theta_1,\ddot\theta_1$ | $a_2,\theta_2,\dot\theta_2,\ddot\theta_2$ | $a_3,\theta_3,\dot\theta_3,\ddot\theta_3$ | $a_4,\theta_4,\dot\theta_4,\ddot\theta_4$ | $a_5,\theta_5,\dot\theta_5$ |
| $t_9$ | $a_1,\theta_1,\dot\theta_1,\ddot\theta_1$ | $a_2,\theta_2,\dot\theta_2,\ddot\theta_2$ | $a_3,\theta_3,\dot\theta_3,\ddot\theta_3$ | $a_4,\theta_4,\dot\theta_4,\ddot\theta_4$ | $a_5,\theta_5$ |
| $t_8$ | $a_1,\theta_1,\dot\theta_1,\ddot\theta_1$ | $a_2,\theta_2,\dot\theta_2,\ddot\theta_2$ | $a_3,\theta_3,\dot\theta_3,\ddot\theta_3$ | $a_4,\theta_4,\dot\theta_4$ | X |
| $t_7$ | $a_1,\theta_1,\dot\theta_1,\ddot\theta_1$ | $a_2,\theta_2,\dot\theta_2,\ddot\theta_2$ | $a_3,\theta_3,\dot\theta_3,\ddot\theta_3$ | $a_4,\theta_4$ | X |
| $t_6$ | $a_1,\theta_1,\dot\theta_1,\ddot\theta_1$ | $a_2,\theta_2,\dot\theta_2,\ddot\theta_2$ | $a_3,\theta_3,\dot\theta_3$ | X | X |
| $t_5$ | $a_1,\theta_1,\dot\theta_1,\ddot\theta_1$ | $a_2,\theta_2,\dot\theta_2,\ddot\theta_2$ | $a_3,\theta_3$ | X | X |
| $t_4$ | $a_1,\theta_1,\dot\theta_1,\ddot\theta_1$ | $a_2,\theta_2,\dot\theta_2$ | X | X | X |
| $t_3$ | $a_1,\theta_1,\dot\theta_1,\ddot\theta_1$ | $a_2,\theta_2$ | X | X | X |
| $t_2$ | $a_1,\theta_1,\dot\theta_1$ | X | X | X | X |
| $t_1$ | $a_1,\theta_1$ | X | X | X | X |

FIG. 6A

EP 1 984 696 B1

EP 1 984 696 B1

$a_1(t_k)$   $M_1(t_k)$   $\theta_1(t_k-1)$   $\theta_1(t_k-2)$   $\Rightarrow$   $\theta_1(t_k)$

$a_1(t_k)$   $\theta_1(t_k)$   $\theta_1(t_k-1)$   $\theta_1(t_k-2)$   $\Rightarrow$   $a_2(t_k)$

$a_2(t_k)$   $M_2(t_k)$   $\theta_2(t_k-1)$   $\theta_2(t_k-2)$   $\Rightarrow$   $\theta_2(t_k)$

$a_2(t_k)$   $\theta_2(t_k)$   $\theta_2(t_k-1)$   $\theta_2(t_k-2)$   $\Rightarrow$   $a_2(t_k)$

## FIG. 6B

## FIG. 7A

RL$_1$

MD$_1$  MD$_2$ — — — — MD$_X$  ML$_1$

RD$_X$

RD$_2$  ML$_2$

RD$_1$  RL$_2$

DEM  ML$_Y$

RL$_Y$

S

RF

E  R

3

G  H  $\vec{L}_1$ — — — — $\vec{L}_Z$

# FIG. 7B

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20030215130 A1
- US 200500883333 A1
- US 6162191 A
- US 6820025 B
- FR 2838185

**Littérature non-brevet citée dans la description**

- **L.Herda ; P.Fua ; R.Pl ankers ; R.Boulic ; D.Thalmann.** *Skeleton-Based Motion Capture for Robust Reconstruction of Human Motion,* Janvier 2000
- **C.Theobalt ; E.Aguiar ; M.Magnor ; H.Theisel ; H-P.Seidel ; MPI Informatik.** *Marker-free Kinematic Skeleton Estimation from Sequences of Volume Data*